# EUROPÄISCHE PATENTSCHRIFT

(11) **EP 2 793 982 B1**
(45) Veröffentlichungstag und Bekanntmachung des Hinweises auf die Patenterteilung: **30.11.2016**
(21) Anmeldenummer: 12812970.7
(22) Anmeldetag: 20.12.2012
(51) Int. Cl.: A61M 5/24, A61M 5/315, B01F 13/00, B01F 15/02, B65D 81/32, B01F 3/08, B01F 3/12

(54) **MEHRKAMMERMISCHBEHÄLTER**
MULTI-CHAMBER MIXING CONTAINER
RÉCIPIENT-MÉLANGEUR À PLUSIEURS COMPARTIMENTS

(30) Priorität: 22.12.2011 EP 11075274
(43) Veröffentlichungstag der Anmeldung: 29.10.2014
(73) Patentinhaber: B. Braun Melsungen AG, 34212 Melsungen (DE)
(72) Erfinder: HARAND, Ralf, 34302 Guxhagen (DE); KRÜGER, Volker, 34329 Nieste (DE); BEINE, Joachim, 34302 Guxhagen (DE); VONHOF, Sandra, 34212 Melsungen (DE)
(74) Vertreter: Arth, Hans-Lothar
(86) Internationale Anmeldenummer: PCT/EP2012/076518
(87) Internationale Veröffentlichungsnummer: WO 2013/092934

(56) Entgegenhaltungen:
- WO-A1-2010/051369
- WO-A1-2010/130682
- GB-A- 787 090
- GB-A- 1 041 311
- US-A- 2 514 575

## Beschreibung

Die Erfindung betrifft einen Mehrkammermischbehälter bestehend aus Stülpbehälter, Entnahmebehälter und verschiedenen optional vorhanden Zwischenbehältern sowie die Anwendung des Mehrkammermischbehälters und Methoden zum Mischen von Flüssigkeiten oder Flüssigkeiten und Feststoffen unter aseptischen Bedingungen in dem Mehrkammermischbehälter.

### Hintergrund

Mehrkammermischsysteme dienen zum Vermischen von zwei oder mehreren Komponenten, vor allem von solchen, die nur kurzfristig miteinander in Kontakt sein sollen. Viele Einzelkomponenten sind in Lösung oder als Mischung mit anderen Komponenten instabil, so dass es vorteilhaft ist, die Komponenten getrennt zu lagern und nur kurz vor der Verwendung der Komponenten miteinander zu mischen oder in Lösung zu bringen. Dies betrifft z.B. Lösungen für medizinische Verwendungen, aber auch industrielle Anwendungen wie die Farbauftragung, Mischung von Chemikalien oder die Verwendung in der Materialherstellung.

In vielen medizinischen, zahnmedizinischen und veterinärmedizinischen Anwendungen ist es notwendig, erst kurz vor der Verwendung bzw. Verabreichung die Komponenten einer Lösung miteinander zu mischen. Diese Präparate und Zubereitungen können flüssige Komponenten aber auch feste Komponenten, z.B. Pulver, umfassen. Viele Wirkstoffe sind in verdünnter Lösung oder überhaupt in gelöster Form nicht stabil, so dass die Mischung und Verabreichung von Lösungen in kurzer Zeit sehr erwünscht ist. Für die Mischung von Flüssigkeiten und/oder Feststoffen sind verschiedene Systeme bekannt. So sind im Stand der Technik vor allem spritzenartige Behälter zum Mischen von zwei oder mehr Komponenten bekannt, wobei die meisten Systeme Zweikammermischsysteme sind, wobei die beiden Kammern fester Bestandteil des Systems sind und nicht beliebig ausgetauscht oder kombiniert werden können. Somit werden die meisten Systeme industriell vorgefertigt. In US2007/0185438 ist eine Vorrichtung offenbart, in der in einer Ampulle mit mehreren Kammern die einzelnen Komponenten einer Mehrkomponentenmischung in verschiedenen Kammern aufbewahrt werden. Für die Mischung der Komponenten werden die Behälter mit einem zur Vorrichtung gehörenden Kolbenelement durchstoßen. Damit wird aus mehreren Kammern ein gesamtes Volumen. Die Ampulle kann in eine spritzenartige Vorrichtung gesetzt werden und unter Verwendung einer angebrachten Nadel direkt als Spritze zur Verabreichung der Mischung verwendet werden. Die Komponenten sind hier durch die Vorfertigung in der Ampulle festgelegt und nicht frei miteinander kombinierbar weder in Hinsicht des Inhalts noch der benötigten Menge oder Konzentration. Die Anmeldung US2010/0121310 betrifft eine Mehrkammerkartusche, in der entlang der Längsachse zwei oder mehrere Kammern angeordnet sind, und zu der weiterhin zwei Kolben gehören, mit deren Hilfe die Komponenten gemischt und das Gemisch durch eine Düse auch verwendet werden kann. Auch die WO2010051369A1 betrifft spritzenartige Vorrichtungen, bei denen ein Zylinder auch in mehrere Anschnitte unterteilt sein kann, welche unerschiedlich befüllt sein können und ein Kolben innerhalb des Zylinders wie bei einer Spritze den Inhalt entleert. Es werden auch Mehrkammerausführungen in WO2010051369A1 offenbart, wobei die einzelnen Kompartimente durch Zwischenwände voneinander getrennt sind, welche durch den Kolben durchstoßen werden können. Diese Zwischenwände werden als dünne Folien bezeichnet, welche sich jedoch nicht vollständig ablösen werden sondern an die innere Zylinderwand legen werden und damit das weitere Vorschieben des Kolbens stoppen werden, Es ist damit offensichtlich, dass die in WO2010051369A1 offenbarten Mehrkammersysteme nicht funktionieren werden, weil der Kolben formschlüssig im Zylinder gleiten muss und die durchtrennten Zwischenwände eine endliche Dicke haben und damit das weitere Vorschieben des Kolbens behindern werden. Durch Aufwendung eines deutlich höheren Drucks mag es möglich sein, den Kolben über die durchtrennten Zwischenwände zu schieben oder die durchtrennten Zwischenwände mit dem Kolben nach vorne zu drücken, wobei dabei die große Gefahr besteht, dass die durch die durchtrennte Zwischenwand aufgebaute Barriere spontan überwunden wird und dann der Inhalt der Spritze mit einem deutlich zu hohen Druck dem Patienten appliziert wird Zudem kommt hinzu, dass bei dem Gleiten eines Kolbens in einem Zylinder entweder Glas als Material verwendet werden kann, welches gute Gleiteigenschaften aufweise, sich dann aber die Zwischenwände technisch nicht einbringen lassen und beim Durchtrennen der Zwischenwände der Zwischenraum zwischen Kolbenaußenfläche und Zylinderinnenwand verstopft wird und der Kolben nicht weiter vorgeschoben werden kann oder bei der Verwendung von Kunststoff als Material für den Zylinder und den Kolben die Zylinderinnenfläche silikonisiert werden muss, um ausreichende Gleiteigenschaften zu gewährleisten. Eine silikonisierte Oberfläche ist jedoch für viele chemische Wirkstoffe und biologische Stoffe unbrauchbar, weil diese Stoffe an silikonisierten Oberflächen denaturiert oder zersetzt werden. Die Ausführungsformen gemäß WO2010051369A1 scheinen daher nur ohne Zwischenwand zu funktionieren, wie auch von den Erfindern der vorliegenden Erfindung nachgewiesen wurde.

GB787090A offenbart einen Einzelbehälter, der in einen Spritzenzylinder eingelegt werden kann und mittels Stülpen entleerbar ist, wobei hier keine individuelle Kombination mehrerer Einzelbehälter vorgesehen ist.

Die offenbarten Vorrichtungen sind jeweils vorgefertigt und an das Vorhandensein von spritzenartigen Kartuschensystemen gebunden. Dabei sind die Kompartimente innerhalb einer vorgefertigten Ampulle oder Kartusche oder Spritze o. ä. durch Membranen oder feste Wände getrennt, jedoch sind nach Herstellung und Befüllung der Kompartimente durch den Hersteller keine Variation der einzelnen Komponenten, also beispielsweise Austausch oder Veränderungen der Reihenfolge in der Anordnung möglich. Weiterhin ist die Anzahl der Kompartimente im Stand der Technik einmal vorgefertigt nicht mehr variierbar. Ein weiterer Nachteil der Ausführungsformen des Standes der Technik ist, dass immer Nadel- und Kolbenartige Teile feste Bestandteile der Vorrichtung sind, die zum Durchstechen und Mischen erforderlich werden.

Aufgabe der vorliegenden Erfindung ist es, eine Vorrichtung bereitzustellen, die zur Mischung von mindestens zwei Komponenten geeignet ist, wobei die Komponenten und auch die Zahl der Komponenten variierbar sein sollen, und die ferner so ausgestaltet sein sollen, dass die Implementierung von Nadeln und Kolben in der Vorrichtung zum Durchmischen der Lösungen nicht notwendig sind. Es soll also ein System bereitgestellt werden, welches es dem Arzt erlaubt, Lösungsmittel und Wirkstoff in Art, Konzentration und Menge frei zu kombinieren, um nach Belieben einen bestimmten Wirkstoff mit einer bestimmten Konzentration in einem bestimmten Lösungsmittel zu verabreichen.

Diese Aufgabe wird durch die Bereitstellung eines Mehrkammermischbehälters gemäß der Ansprüche 1, 7 und 8 und der Methode gemäß Anspruch 15 gelöst. Weitere vorteilhafte Ausgestaltungen, Aspekte und Details der Erfindung ergeben sich aus den abhängigen Ansprüchen, der Beschreibung, den Beispielen und den Figuren.

### Beschreibung der Erfindung

Überraschend wurde gefunden, dass der erfindungsgemäße Mehrkammermischbehälter zur Bereitstellung und Mischung der Komponenten die gestellte Aufgabe löst. Der erfindungsgemäße Mehrkammermischbehälter dient zur getrennten Lagerung von mindestens einem Lösungsmittel und einer Flüssigkeit oder einem Wirkstoff, der beispielsweise im lyophilisierten, sprühgetrockneten oder gefriergetrockneten Zustand stabiler als im gelösten Zustand ist, so dass eine getrennte Lagerung von Lösungsmittel und Wirkstoff im Mehrkammermischbehälter erfolgen kann und die aseptische Durchmischung kurz vor der Anwendung ebenfalls in dem erfindungsgemäßen Mehrkammermischbehälter durchgeführt werden kann.

Zu den Wirkstoffen, welche vorzugsweise für parenterale Anwendungen in dem Mehrkammermischbehälter aufbewahrt werden können, zählen beispielsweise Antibiotika, Analgetika, antientzündliche Wirkstoffe, Steroide, antiproliferative, immunsuppressive, fungizide, zytostatische, antimigrative, antiphlogistische, zytotoxische, antiangiogene oder/und antithrombotische Wirkstoffe. Flüssigkeiten zur Lösung oder Mischung umfassen aqua bidest., isotonische Natriumchloridlösung, verschiedene andere isotonische Salzlösungen oder physiologisch verträgliche Puffer.

Bei dem Begriff "Flüssigkeit" wie hierin verwendet, handelt es sich nicht nur um Lösungsmittel für einen Feststoff, ein Feststoffgemisch, ein Gel, eine Paste, einen anderen flüssigen Stoff oder ein Flüssigkeitsgemisch, sondern auch um Lösungen von festen Stoffen, Emulsionen oder Dispersionen oder Zweiphasenflüssigkeitsgemischen.

Der erfindungsgemäße Mehrkammermischbehälter besteht in der einfachsten Ausführungsform aus dem Stülpbehälter und einem Entnahmebehälter, welche aseptisch miteinander verbunden sind. Das Lösungsmittel befindet sich in dem Stülpbehälter und der oder die Wirkstoffe in fester, gelartiger oder flüssiger Form in dem Entnahmebehälter. Der Wirkstoff oder die Wirkstoffkombination in dem Entnahmebehälter ist vorzugsweise ein Feststoff, der sich als Feststoff länger lagern lässt als in Lösung, so dass die Wirkstofflösung erst kurz vor der Anwendung hergestellt werden soll. Grundsätzlich ist der Mehrkammermischbehälter für die Lagerung von Wirkstoffen geeignet, welche ohne Lösungsmittel länger stabil sind als in einem Lösungsmittel, wobei das Lösungsmittel auch Puffer einschließt. Ganz besonders ist der Mehrkammermischbehälter aber dafür gedacht, dem Arzt die Kombination der Einzelbehälter zu ermöglichen, so dass ein Arzt einen Behälter mit einem bestimmten Wirkstoff in einer bestimmten Menge auswählen kann und diesen mit einem Behälter mit einem bestimmten Lösungsmittel in einer bestimmten Menge kombinieren kann, um dadurch eine individuelle Mischung eines bestimmten Wirkstoffs in einer bestimmten Konzentration in einer bestimmten Menge eines bestimmten Lösungsmittels herzustellen.

Ist es notwendig, mehrere Wirkstoffe oder mehrere Lösungsmittel oder einen flüssigen Wirkstoff und einen festen Wirkstoff getrennt vom Lösungsmittel zu lagern, so kann der erfindungsgemäße Mehrkammermischbehälter neben dem Stülpbehälter und dem Entnahmebehälter auch noch einen, zwei oder mehrere Zwischenbehälter aufweisen, welche sich zwischen dem Stülpbehälter und dem Entnahmebehälter befinden und vorzugsweise zur Aufnahme jeweils einer Komponente dienen.

Stülpbehälter, Entnahmebehälter und Zwischenbehälter werden generisch auch als Komponentenbehälter bezeichnet.

Die Komponentenbehälter sind verschlossen, wobei der Verschluss der Komponentenbehälter vorzugsweise durch eine Versiegelung mit einer Siegelfolie geschieht. Zweck der Komponentenbehälter ist es, den Inhalt steril und keimfrei zu halten. Die erfindungsgemäßen Komponentenbehälter weisen weiterhin vorzugsweise mindestens eine Zentriernut, Vertiefung, eine Wulst, ein Gewinde oder auch einen Zapfen in der Seitenfläche auf. Diese sind vorteilhaft, weil bei einer Anordnung des oder der Komponentenbehälter in einer Vorrichtung zur Durchmischung ein mechanischer Halt des oder der Behälter gewährleistet werden kann.

Es werden also alle Komponentenbehälter, d.h. zwei (Bi-System), drei (Tri-System), vier (Quad-System) oder ganz allgemein mehrere Komponentenbehälter (Multisystem) formschlüssig, kraftschlüssig oder stoffschlüssig und aseptisch miteinander verbunden. Die Gestaltung der Komponentenbehälter erlaubt außerdem das Verbinden mehrerer Komponentenbehälter zu einem Bi-, Tri- oder Quad-System, um mehrere über einen längeren Zeitraum inkompatible Inhaltsstoffe getrennt zu lagern und kurz vor der Anwendung miteinander zu mischen.

Die vorliegende Erfindung betrifft daher nicht nur fertig zusammengesetzte Mehrkammermischbehälter sondern auch einen Kit aus mindestens einem Stülpbehälter, mindestens einem Entnahmebehälter und optional mindestens einem Zwischenbehälter, welche individuell miteinander kombiniert werden können.

Ein Bi-System besteht dabei aus einem Stülpbehälter und einem Entnahmebehälter, ein Tri-System aus einem Stülpbehälter, einem Zwischenbehälter und einem Entnahmebehälter und ein Quad-System aus einem Stülpbehälter, einem ersten Zwischenbehälter, einem zweiten Zwischenbehälter und einem Entnahmebehälter. Jeder Komponentenbehälter kann dabei eine Komponente oder auch ein Komponentengemisch der später herzustellenden Lösung aufnehmen, getrennt von den anderen Komponenten aufbewahren sowie steril und keimfrei halten. Auch die Verbindung der Komponentenbehälter miteinander erfolgt aseptisch.

Optional können die Komponentenbehälter jeweils einen Fügerand oder ein Gewinde aufweisen, der das Zusammenfügen eines Komponentenbehälters mit einem oder zwei weiteren Komponentenbehältern ermöglicht, der Verbindung einen mechanischen Halt vermittelt, und den Verschluss der Verbindung nach außen erleichtert.

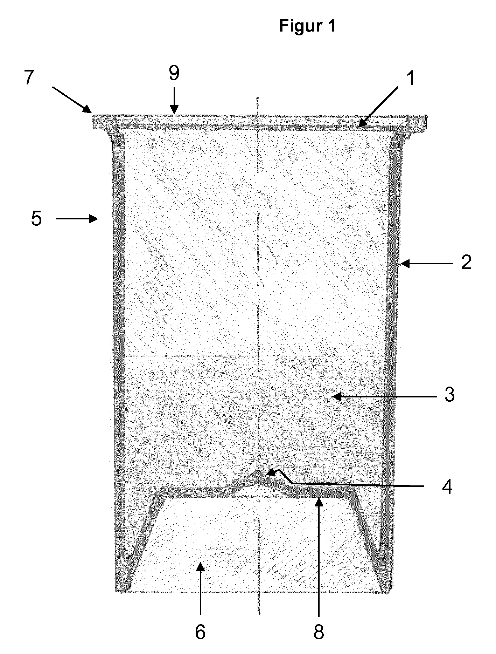

Zur Erfindung gehören verschiedene Ausführungsformen der Komponentenbehälter. Der Stülpbehälter ist vorzugsweise ein Flüssigkeitsbehälter. Ein erfindungsgemäßer Stülpbehälter weist eine stülpbare Seitenwandung, eine versiegelbare Stirnseite, weiterhin vorzugsweise eine innenliegende Nase oder Wölbung zum Durchdrücken der Siegelfolie, die durch Umstülpen der Seitenwandung axial bewegt werden kann, und vorzugsweise mindestens eine Zentriernut oder Vertiefung in der Wandung auf (Figur 1). Die stülpbare Seitenwandung ist vorteilhaft, weil dadurch ein Eindrücken einer Behälterfläche durch Druck von außen ermöglicht wird. Die einstülpbaren Teile der Seitenwandung sind vorzugsweise weicher, also schwächer in der Materialbeschaffenheit, um ein leichtes Eindrücken zu ermöglichen, ohne dass das Material beschädigt wird oder ein zu hoher Kraftaufwand notwendig wird. Das Eindrücken bzw. Einstülpen wird dabei bevorzugt von sogenannten Rastlinien unterstützt, um ein leichteres Eindrücken zu ermöglichen. Die Rastlinien zeichnen sich durch einen Übergang zu einer stärkeren oder schwächeren Materialbeschaffenheit aus, die die Stülpeigenschaften verändert wie z.B. eine Verjüngung oder Verdickung im Material oder der Wand, eine Sollfaltstelle oder Sollfaltlinie oder eine gezielte Schwachstelle im Material bzw. der Wand.

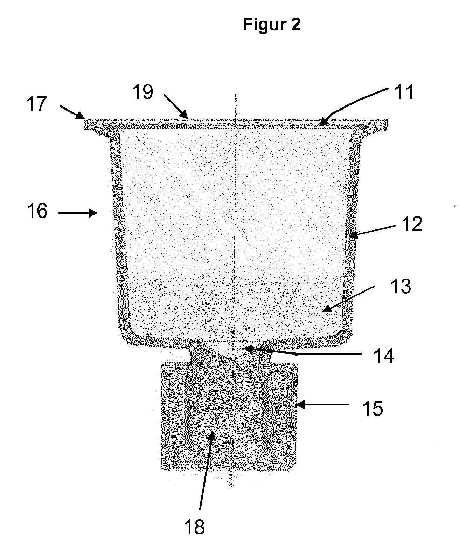

Ein weiterer Komponentenbehälter ist der Entnahmebehälter. Der Entnahmebehälter wird in seiner äußeren Form durch Stirnseite, Seitenwandung und Verschlussseite charakterisiert. Die Stirnseite ist wie beim Stülpbehälter durch eine durchdrückbare Folie versiegelt. Der Entnahmebehälter kann dabei eine Flüssigkeit oder einen festen Stoff enthalten. Feste Stoffe sind bevorzugt pulverförmig, ebenfalls bevorzugt lyophilisiert und weiterhin bevorzugt gefriergetrocknet. Die Form des Entnahmebehälters ist dabei vorzugsweise der des Stülpbehälters angepasst, d.h. der Durchmesser und die Form der Stirnseite entspricht der des Stülpbehälters, wodurch eine dichte, also stoffschlüssige Verbindung zwischen beiden Komponentenbehältern gewährleistet werden kann. Der Entnahmebehälter weist in seiner Verschlussseite vorzugsweise ein Verschlusssystem auf, durch das der Entnahmebehälter hermetisch verschlossen wird, und der Inhalt mit einer Kanüle entnommen werden kann (Figur 2). Alternativ kann der Inhalt des Entnahmebehälters bzw. des Mehrkammermischbehälters durch einen nadelfreien Zugang abgeleitet werden.

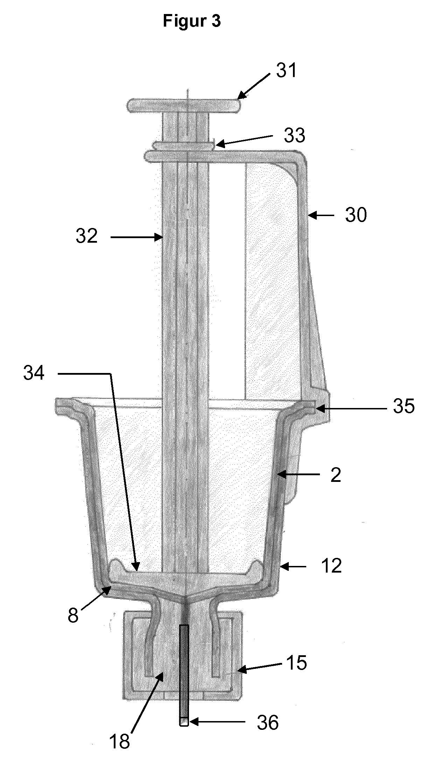

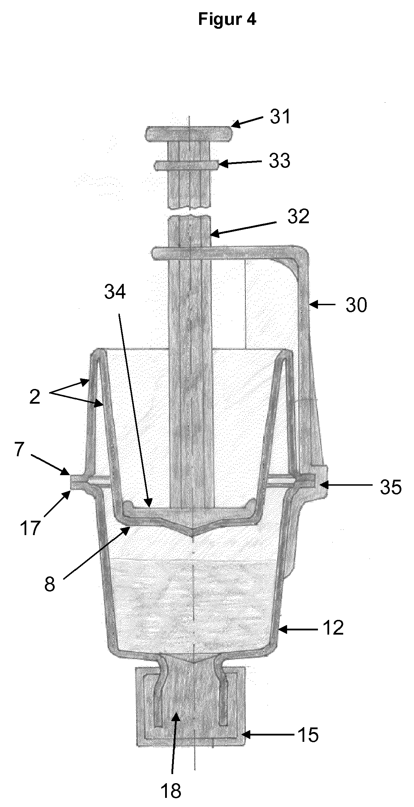

Zudem wird hierin eine Vorrichtung offenbart, in die der Mehrkammermischbehälter eingelegt und vorzugsweise durch entsprechende Aussparungen in der Vorrichtung fixiert werden kann. Die Vorrichtung ist halbschalenförmig und weist einen in axialer Richtung verschiebbaren Ausdrückstempel auf. Diese Vorrichtung ist ein karpulenartiges System, das zur Durchmischung der Inhaltsstoffe der Komponentenbehälter verwendet wird (Figur 3). Die Vorrichtung ist vorzugsweise als halbschalenförmige Aufnahme mit einer oder mehreren Zentriernuten in der Verbindungsebene der Komponentenbehälter ausgeführt, die den Mehrkammermischbehälter zentrieren und in seiner Lage stabilisieren. Die Vorrichtung für einen Mehrkammermischbehälter mit einem, zwei oder mehr Zwischenbehältern ist prinzipiell ähnlich aufgebaut, lediglich Länge und Anzahl der vorzugsweise vorhandenen Zentriernuten sind an die jeweilige Konfiguration angepasst. Durch Ausübung von Druck durch den Ausdrückstempel auf den Boden des Stülpbehälters und eine nachfolgende Bewegung des Ausdrückstempels in Richtung Siegelfolie, was auch in Richtung des Entnahmebehälters ist, wird der Stülpbehälter eingedrückt und seine Wandung stülpt sich nach innen. Mit fortschreitender Bewegung des Ausdrückstempels in Richtung Entnahmebehälter durchstößt der Boden des Stülpbehälters die Siegelfolie des Stülpbehälters und danach die Siegelfolie des Entnahmebehälters und die Flüssigkeit aus dem Stülpbehälter kann in den Entnahmebehälter übertreten und den Stoff lösen (Figur 4). Die so frisch zubereitete Lösung kann aus dem Entnahmebehälter durch einen Entnahmebereich wie z.B. einen Stopfen oder ein Septum entnommen werden. Zum vollständigen Entleeren des Mehrkammermischbehälters kann der Stülpbehälter vollständig in den Entnahmebehälter gestülpt werden bis der Boden des Stülpbehälters am Entnahmebereich des Entnahmebehälters anliegt (Figur 3).

### Ausführliche Beschreibung der Erfindung

Überraschend wurde gefunden, dass ein Mehrkammermischbehälter zur getrennten Lagerung und Mischung der enthaltenen Komponenten die gestellte Aufgabe löst, wobei der erfindungsgemäße Mehrkammermischbehälter aus einem Stülpbehälter und einem Entnahmebehälter gefüllt mit einer Flüssigkeit oder einem Feststoff besteht oder diese umfasst, wobei Stülpbehälter und Entnahmebehälter dichtend miteinander verbunden sind, der Entnahmebehälter auf der Oberseite mit einer durchdrückbaren Siegelfolie verschlossen ist und auf der Unterseite einen Entnahmebereich aufweist.

Der erfindungsgemäße Mehrkammermischbehälter besteht in der einfachsten Ausführungsform somit aus einem Stülpbehälter und einem Entnahmebehälter, wobei der Stülpbehälter immer mit einer Flüssigkeit gefüllt ist. Der Entnahmebehälter enthält vorzugsweise einen Feststoff oder ein Feststoffgemisch, kann aber auch eine weitere Flüssigkeit oder ein Flüssigkeitsgemisch oder eine Lösung von einem oder mehreren Stoffen in einem Lösungsmittel enthalten. Vorzugsweise ist die im Entnahmebehälter enthaltene Komponente länger stabil, wenn diese getrennt von der Flüssigkeit im Stülpbehälter gelagert wird als wenn die im Entnahmebehälter enthaltene Komponente in der Flüssigkeit gelöst ist. Durch Stülpen des Stülpbehälters zumindest teilweise in den Entnahmebehälter erfolgt die Durchtrennung der Unterteilung zwischen beiden Komponentenbehältern und die Komponente enthalten im Entnahmebehälter kann in der Flüssigkeit enthalten in dem Stülpbehälter gelöst werden.

Somit betrifft die vorliegende Erfindung einen Mehrkammermischbehälter umfassend oder bestehend aus einem Stülpbehälter und einem Entnahmebehälter, wobei der Stülpbehälter mit einer Flüssigkeit gefüllt ist und stirnseitig mit einer durchdrückbaren Siegelfolie verschlossen ist und dessen Boden in Richtung der Siegelfolie drückbar ist und die Wandung nach innen stülpbar ist und der Entnahmebehälter mit einer Flüssigkeit oder einem Feststoff gefüllt ist und auf der Oberseite mit einer durchdrückbaren Siegelfolie verschlossen ist und auf der Unterseite einen Entnahmebereich aufweist und Stülpbehälter und Entnahmebehälter dichtend miteinander verbunden sind. Als Entnahmebereich kann ein Stopfen, Septum, Ventil, Verschluss, Schraubverschluss oder andere Verschlüsse zum Verbinden mit einer Kanüle oder einem Schlauch dienen.

Vorzugsweise ist die mit der durchdrückbaren Siegelfolie verschlossene Stirnseite des Stülpbehälters mit der mit der durchdrückbaren Siegelfolie verschlossenen Oberseite des Entnahmebehälters dichtend verbunden.

Zudem ist bevorzugt, wenn die Außenseite des Stülpbehälters in Höhe der Siegelfolie eine Nut, eine Wulst, ein Gewinde oder eine Einbuchtung aufweist und/oder der Entnahmebehälter in Höhe der Siegelfolie eine Nut, eine Wulst, ein Gewinde oder eine Einbuchtung aufweist. Diese Nut, Wulst, Gewinde oder Einbuchtung können zum einem zur Fixierung des Mehrkammermischbehälters in der Vorrichtung zur Aufnahme des Mehrkammermischbehälters dienen als auch zur dichtenden Verbindung der beiden Komponentenbehälter miteinander. Es versteht sich von selbst, dass bei einer dichtenden Verbindung zweier Komponentenbehälter miteinander die Nut des einen Komponentenbehälters zu der Nut des anderen oder die Wulst des einen Komponentenbehälters zu der Wulst des anderen oder das Gewinde des einen Komponentenbehälters zu dem Gewinde des anderen oder die Einbuchtung des einen Komponentenbehälters zu der Ausbuchtung des anderen Komponentenbehälters formschlüssig passen müssen, damit eine dichtende Verbindung eventuell nach einem Klebe-, Strahlungs- oder Wärmebehandlungsschritt hergestellt werden kann.

In einer weiteren Ausführungsform besteht der Mehrkammermischbehälter aus dem erfindungsgemäßen Stülpbehälter und dem Entnahmebehälter und einem zwischen Stülpbehälter und Entnahmebehälter befindlichen Zwischenbehälter gefüllt mit einer Flüssigkeit oder einem Feststoff, wobei der Zwischenbehälter an der Oberseite und an der Unterseite mit einer durchdrückbaren Siegelfolie verschlossen ist und der Zwischenbehälter dichtend mit dem Stülpbehälter und dichtend mit dem Entnahmebehälter verbunden ist.

In einer anderen Ausführungsform besteht der Mehrkammermischbehälter aus dem Stülpbehälter und dem Entnahmebehälter und zwei zwischen Stülpbehälter und Entnahmebehälter befindlichen Zwischenbehältern jeweils gefüllt mit einer Flüssigkeit oder einem Feststoff, wobei beide Zwischenbehälter an der Oberseite und an der Unterseite mit einer durchdrückbaren Siegelfolie verschlossen sind und beide Zwischenbehälter dichtend miteinander verbunden sind und der eine Zwischenbehälter dichtend mit dem Stülpbehälter und der andere Zwischenbehälter dichtend mit dem Entnahmebehälter verbunden ist.

Der erfindungsgemäße Mehrkammermischbehälter besteht also immer aus dem Stülpbehälter, der eine Flüssigkeit enthält und dem Entnahmebehälter, der eine feste oder flüssige Komponente enthält und optional einem, zwei oder mehr zwischen Stülpbehälter und Entnahmebehälter liegenden Zwischenbehältern, wobei jeder Komponentenbehälter mit dem anliegenden Komponentenbehälter dichtend verbunden ist, d.h. bevorzugt aseptisch verschweißt oder verklebt ist und die miteinander dichtend verbundenen Flächen der Komponentenbehälter mit einer Siegelfolie verschlossen sind. Dieses Aufbauprinzip ermöglicht die Herstellung und Befüllung und Versiegelung der einzelnen Behälter unter aseptischen Bedingungen, welche dann erst zu dem erfindungsgemäßen Mehrkammermischbehälter zusammengesetzt werden.
Der Begriff "dichtend verbunden" oder "dichtend verbindbar" bezeichnet die Eigenschaft, dass zwei Komponentenbehälter so über eine Schweißverbindung, Klebeverbindung, Gewinde oder andersartig verbunden oder verbindbar sind, dass bei dem zum Entleeren des Mehrkammermischbehälters aufzubauenden Drucks kein Inhalt aus dem Mehrkammermischbehälter durch diese dichtende Verbindung entweichen kann.

Ferner betrifft die vorliegende Erfindung einen Kit umfassend mindestens einen Stülpbehälter, der stirnseitig mit einer durchdrückbaren Siegelfolie verschlossen ist und dessen Boden in Richtung der Siegelfolie drückbar ist und die Wandung nach innen stülpbar ist und mindestens einen Entnahmebehälter, der auf der Oberseite mit einer durchdrückbaren Siegelfolie verschlossen ist und auf der Unterseite einen Entnahmebereich aufweist, wobei Stülpbehälter und Entnahmebehälter dichtend miteinander verbindbar sind.

Vorzugsweise ist der mindestens eine Stülpbehälter mit einer Flüssigkeit wie z.B. einem Lösungsmittel, einem Puffer oder einer Wirkstofflösung gefüllt und noch bevorzugter vollständig gefüllt. Dieser Kit kann zudem noch mindestens einen Zwischenbehälter umfassen, der an der Oberseite und an der Unterseite mit einer durchdrückbaren Siegelfolie verschlossen ist, wobei der Zwischenbehälter dichtend mit dem Stülpbehälter und dichtend mit dem Entnahmebehälter verbindbar ist.

Alle Komponentenbehälter in diesem Kit sind vorzugsweise vorgefüllt und können beliebig miteinander kombiniert werden. Ein erfindungsgemäßer Kit kann somit mehrere Stülpbehälter umfassen, welche beispielsweise mit unterschiedlichen Puffern befüllt sind sowie Stülpbehälter mit unterschiedlichen Mengen an beispielsweise physiologischer Kochsalzlösung. Die Zwischenbehälter des Kit können beispielsweise Lösungen oder Feststoffe von Vitaminen, Salzen oder Nährungsergänzungsmittels enthalten in unterschiedlichen Zusammensetzungen und Mengen enthalten und die Entnahmebehälter können einen Wirkstoff oder ein Wirkstoffgemisch als Feststoff oder als Lösung enthalten. Ein solcher Kit ermöglicht es einem Arzt beispielsweise bei einer Grippeimpfung einen Stülpbehälter mit einer 0,5M Kochsalzlösung, einen Zwischenbehälter mit 0,5g Vitamin C, Folsäure und einigen B-Vitaminen und einen Entnahmebehälter mit einem bestimmten Antibiotikum in einer bestimmten Mengen je nach Geschlecht, Alter und Gewicht des Patienten auszuwählen und individuell zu kombinieren und nach Vereinigung der Komponenten im Mehrkammermischbehälter als homogene Lösung zu verabreichen.

Die Herstellung und vorzugsweise aseptische Herstellung einer Lösung der Komponenten kann gemäß folgender Methode erfolgen, so dass die vorliegende Erfindung auch auf eine Methode zur aseptischen Herstellung einer Lösung aus mindestens zwei Komponenten gerichtet ist, charakterisiert durch das Einlegen eines erfindungsgemäßen Mehrkammermischbehälters in eine Vorrichtung umfassend eine halbschalenförmige Aufnahme für den Mehrkammermischbehälter, Haltemittel für den Mehrkammermischbehälter und einen in axialer Richtung beweglichen Ausdrückstempel, Ausübung von Druck durch den Ausdrückstempel auf den Boden des Stülpbehälters, Drücken des Bodens des Stülpbehälters in Richtung des Entnahmebehälters, wobei sich die Wandung des Stülpbehälters kontinuierlich nach innen stülpt und mit forschreitendem Drücken die Siegelfolien durchdrückt werden und Vermischung der mindestens zwei Komponenten.

In diesem Kit oder in zusammengesetzter Form als Mehrkammermischbehälter ist der Stülpbehälter durch einen nach innen eindrückbaren Boden, eine gegenüberliegende mit einer Siegelfolie verschlossene Fläche und eine nach innen stülpbare Wandung gekennzeichnet. Der Zwischenbehälter zeichnet sich durch eine stabile, d.h. nicht stülpbare, bis zu einem Teil der Höhe des Behälters stülpbare oder vollständig stülpbare Wandung aus und einer mit einer Siegelfolie verschlossenen Oberseite sowie einer mit einer Siegelfolie verschlossenen Unterseite. Der Entnahmebehälter zeichnet sich durch einen Bereich, den Entnahmebereich, aus, durch den die in dem Mehrkammermischbehälter hergestellte Lösung vorzugsweise durch eine Nadel, Kanüle oder auch nadelfrei entnommen werden kann, eine gegenüberliegende mit einer Siegelfolie verschlossene Seite und eine stabile, d.h. nicht stülpbare Wandung. Zudem weisen alle Komponentenbehälter eine gleichmäßige Geometrie ihrer Querschnittsfläche auf und sind vorzugsweise rund oder oval, so dass ein Einstülpen des Stülpbehälters möglichst bequem erfolgen kann. Zudem weisen die Komponentenbehälter vorzugsweise denselben Innenradius auf, damit diese passend zusammengesetzt werden können und die Verbindungslinie oder der Verbindungsrand aufeinander passen. Der Mehrkammermischbehälter ist somit vorzugsweise zylinderförmig mit einem flachen oder nach innen gewölbten Boden, nämlich dem Boden des Stülpbehälters und einem kopfseitigen Entnahmebereich in Form einer Kappe, Bördelkappe, Schraubkappe, Stopfen oder der gleichen, durch Siegelfolien voneinander getrennten Kompartimenten und einem länglichen vorzugsweise zylinderförmigen Korpus.

Die Komponentenbehälter oder Stülpbehälter und Entnahmebehälter werden so miteinander verbunden, dass die Siegelfolien in kurzem Abstand aufeinander liegen. Zur form-, kraft- und/oder stoffschlüssigen, d.h. dichtenden und aseptischen Verbindung werden die Komponentenbehälter vorzugsweise durch Stecken, Schrauben, Kleben, Einpressen, besonders bevorzugt aber durch Schweißen miteinander verbunden, damit beim Vermischen der Inhalte keine Undichtigkeit auftritt und der aseptische Inhalt weiterhin steril und keimfrei bleibt. Die Verbindung wird unabhängig vom Fügeverfahren aseptisch also unter sterilen Bedingungen durchgeführt, damit keine Keime in den Zwischenraum zwischen den Siegelfolien gelangen. Der optional vorhandene Fügerand gibt der Verbindung einen mechanischen Halt und trägt weiter zum Abdichten der Verbindung bei. Möglich ist auch ein Verschluss des Komponentenbehälters durch vollständiges Verschweißen bzw. Verschmelzen des Komponentenbehältermaterials oder eines Überzugs mit einem flüssigkeits-undurchlässigen Material wie Plastik, einem korrosionsbeständigen Metall oder einem Polymer.

Der durch die Verbindung der Komponentenbehälter entstandene Mehrkammermischbehälter weist vorzugsweise eine zylindrische Form auf, die durch die Form der Komponentenbehälter definiert wird. In Form und Durchmesser entspricht der Mehrkammermischbehälter also Stülpbehälter und Entnahmebehälter als auch dem optionalen Zwischenbehälter.

Die sich gegenüberliegenden Siegelfolien von Stülp- und Entnahmebehälter im Bi-System oder von Stülp-, Zwischen- und Entnahmebehälter im Tri-System, Quad-Sytem oder Multi-System werden zur Vermischung der Inhalte im Sinne der Erfindung von dem Boden des Stülpbehälters oder der Durchdrücknase des Bodens des Stülpbehälters nacheinander durchdrückt. Durch mechanischen Druck auf den Boden des Stülpbehälters wird dabei der Boden oder die Durchdrücknase auf der Bodeninnenseite in Richtung der Siegelfolien gedrückt, die durch den Druck einreißen. Dabei wird zuerst die Siegelfolie des Stülpbehälters und dann die Siegelfolie des Entnahmebehälters oder sofern vorhanden die Siegelfolie des Zwischenbehälters durchstoßen. Durch die entstandenen Öffnungen kommen die Inhalte der Komponentenbehälter miteinander in Kontakt, werden die Flüssigkeiten miteinander gemischt bzw. ein im Entnahmebehälter befindlicher Feststoff in der Flüssigkeit gelöst, welche im Stülpbehälter enthalten war. Der Druck, der für ein Durchdrücken oder Einreißen der Siegelfolien und damit ein Einströmen bzw. Überströmen von Flüssigkeit vom Stülpbehälter in den Entnahmebehälter sorgt, wird durch den auf den Boden des Stülpbehälters ausgeübten mechanischen Druck erzeugt (Figur 3).

Je weiter der Stülpbehälter eingestülpt wird, desto größer wird der Druck im Mehrkammermischbehälter. Dieser erhöhte Druck kann gemindert werden, indem die erhaltene Lösung entweder mit einer Nadel oder auch nadelfrei über den Entnahmebereich des Entnahmebehälters entnommen wird. Bei einer Entnahme mittels Nadel oder Kanüle wird die erhaltene Lösung vorzugsweise in eine Spritze oder eine Volumenmesseinrichtung aufgezogen. Bei einer nadelfreien Entnahme der erhaltenen Lösung erfolgt beispielsweise eine Überführung der Lösung in einen Tropftrichter oder Tropfbeutel worin sich auch schon eine andere Flüssigkeit befinden kann. Soll hingegen die hergestellte Lösung noch nicht entnommen werden, so kann auch der entstehende Druck über eine Nadel, Kanüle oder ein am Entnahmebehälter befindlichen Überdruckventil abgelassen werden.

Der erfindungsgemäße Stülpbehälter ist ein Komponentenbehälter zur Aufnahme einer Flüssigkeit, der stirnseitig mit einer durchdrückbaren Siegelfolie verschlossen ist und dessen Boden in Richtung der Siegelfolie drückbar ist und die Wandung nach innen stülpbar ist. Vorzugsweise ist der Stülpbehälter vollständig gefüllt, d.h. ohne Gaseinschluss über der Flüssigkeit.

Der Stülpbehälter wird in seiner äußeren Form durch Boden, Wandung und Stirnseite charakterisiert. Der Begriff der Stülpbarkeit wird durch die Veränderung einer Form definiert. Die stülpbare Wandung wird dabei mit dem Boden des Stülpbehälters nach innen gedrückt, so dass vorzugsweise die Innenseite der gestülpten Wandung an der Innenseite der noch nicht gestülpten Wandung entlang gleitet und der Stülpbehälter während des Stülpvorgangs an Höhe verliert (Figur 4). Besteht der Mehrkammermischbehälter nur aus einem Stülpbehälter und einem Entnahmebehälter so kann bei vollständigem Stülpen des Stülpbehälters die Innenseite der Wandung des Stülpbehälters an der Innenseite der Wandung des Entnahmebehälters anliegen. Weisen Stülpbehälter und Entnahmebehälter eine gleiche Höhe auf, so lässt sich der Stülpbehälter vollständig in den Entnahmebehälter stülpen und der Boden des Stülpbehälters liegt am oder nahe dem Entnahmebereich des Entnahmebehälters (Figur 3).

Der erfindungsgemäße Stülpbehälter ist also dadurch charakterisiert, dass bei entsprechender Wirkung einer Kraft auf seinen Boden die Wandung nach innen gedrückt wird. Mit anderen Worten kann das Stülpen auch als durch Eindrücken bewirkte plastische Verformung des erfindungsgemäßen Stülpbehälters bezeichnet werden. Die stülpbare Wandung ist vorteilhaft, weil dadurch eine Formveränderung durch Eindrücken des Stülpbehälters in den Entnahmebehälter oder in den oder die Zwischenbehälter und den Entnahmebehälter bewirkt wird. Der Druck von außen bedeutet mechanisches Drücken bevorzugt auf die Mitte des Bodens des Stülpbehälters.

Stülpbar gemäß vorliegender Erfindung ist somit die Wandung des Stülpbehälters zumindest so weit, bis die Siegelfolie des Entnahmebehälters durchstoßen wird. Bei einem Mehrkammermischbehälter aus Stülpbehälter und Entnahmebehälter kann der Stülpbehälter vorzugsweise bis zur Verbindungsebene beider Komponentenbehälter gestülpt werden, sofern der Entnahmebehälter die gleiche oder eine größere Höhe hat als der Stülpbehälter. Befindet sich zwischen Stülpbehälter und Entnahmebehälter noch ein Zwischenbehälter, so können die einzelnen Komponentenbehälter dieselbe oder verschiedene Höhen aufweisen. Haben alle drei Komponentenbehälter dieselbe Höhe, dann ist bevorzugt, dass eine Stülpung bis zur mittleren Höhe des Zwischenbehälters erfolgen kann, so dass der Stülpbehälter vollständig in den Entnahmebehälter und der Zwischenbehälter bis zur Hälfte in sich selbst gestülpt werden kann. Besitzen hingegen Entnahmebehälter und Zwischenbehälter zusammen dieselbe Höhe wie der Stülpbehälter, so braucht nur der Stülpbehälter stülpbar ausgestaltet zu sein, damit der Stülpbehälter vollständig in den Entnahmebehälter als auch den Zwischenbehälter gestülpt werden kann. Hat hingegen der Entnahmebehälter dieselbe Höhe wie Stülpbehälter und Zwischenbehälter zusammen, so sollten Stülpbehälter und Zwischenbehälter stülpbar ausgestaltet sein, damit Stülpbehälter als auch Zwischenbehälter in den Entnahmebehälter gestülpt werden können. Natürlich sind auch beliebige andere Höhen der Komponentenbehälter möglich. Vorzugsweise sollte aber ein weitgehendes Stülpen oder Einstülpen möglich sein, wobei der Mehrkammermischbehälter nach vollständigem Stülpen oder Einstülpen ca. 50% seiner Höhe verliert.

Besteht der Mehrkammermischbehälter aus einem Stülpbehälter, einem Entnahmebehälter und zwei Zwischenbehältern, so sind entsprechend auch wieder unterschiedliche Höhen der Komponentenbehälter möglich. Weisen alle vier Komponentenbehälter dieselbe Höhe auf, dann ist bevorzugt, wenn der Stülpbehälter und der am Stülpbehälter angrenzende erste Zwischenbehälter stülpbar sind, so dass bei vollständigem Stülpen oder Einstülpen die Innenseite der Wandung des Stülpbehälters an der Innenseite der Wandung des Entnahmebehälter und die Innenseite der Wandung des ersten Zwischenbehälters an der Innenseite der Wandung des zweiten Zwischenbehälters anliegt. Haben hingegen Entnahmebehälter, erster Zwischenbehälter und zweiter Zwischenbehälter zusammen dieselbe Höhe wie der Stülpbehälter, dann braucht nur der Stülpbehälter stülpbar ausgestaltet zu sein, damit dieser in den Entnahmebehälter und die beiden Zwischenbehälter gestülpt werden kann. Haben Stülpbehälter und die beiden Zwischenbehälter zusammen dieselbe Höhe wie der Entnahmebehälter, dann ist bevorzugt, wenn Stülpbehälter und beide Zwischenbehälter stülpbar ausgestaltet sind, damit diese drei Komponentenbehälter in den Entnahmebehälter gestülpt werden können. Es versteht sich für jeden Fachmann, dass die vier Komponentenbehälter auch andere beliebige Höhen haben können und eine Stülpbarkeit vorzugsweise vom Boden bis zur Mitte des Mehrkammermischbehälters gegeben sein sollte, damit der untere Teil des Mehrkammermischbehälters in den oberen Teil des Mehrkammermischbehälters gestülpt werden kann.

Somit kann es erforderlich sein, einen Komponentenbehälter so auszugestalten, dass nicht seine gesamte Wandung sondern die Wandung nur bis zu einer bestimmten Höhe stülpbar ist.

Die Stülpbarkeit wird in erster Linie durch die Materialbeschaffenheit definiert. Dabei bedeuten besonders feste Materialien keine oder geringe Stülpbarkeit, so dass eine Materialbeschaffenheit bevorzugt ist, die ein leichtes Eindrücken und/oder Umfalten ermöglicht, ohne dass das Material beschädigt wird oder ein zu hoher Kraftaufwand notwendig ist. Jedoch darf das Material nicht so weich sein, dass die Form des flüssigkeitsenthaltenden Stülpbehälters nicht stabil ist oder eine mechanische Beschädigung des Stülpbehälters möglich ist. Der Behälter kann aus einem beliebigen Material bestehen, dass sich zur Aufbewahrung einer Flüssigkeit eignet. Bevorzugte Materialien sind dabei Metall, eine Metalllegierung, Gummi oder ein beliebiges flüssigkeitsundurchlässiges Polymer, wobei Polymere und Gummi bevorzugter sind, und besonders bevorzugt Polymere, und ganz besonders Plastik. Dabei können verschiedene Steifigkeiten des Kunststoffs die Stülpbarkeit beeinflussen. So ist Weichplastik, also besonders biegsamer Kunststoff wie beispielsweise Weich-PVC oder Polyolefine, z.B. Polyethylen, für die Herstellung des stülpbaren Teils besonders bevorzugt, und Hartplastik bevorzugt für den Teil, der nicht gestülpt werden soll. Das Eindrücken bzw. das Einstülpen kann dabei durch sogenannte Rastlinien unterstützt aber auch begrenzt werden. Die Rastlinien, welche die Stülpbarkeit begrenzen sollen, zeichnen sich durch einen Übergang zu einer stärkeren Materialbeschaffenheit aus, die ein weiteres einfaches Einstülpen verhindern, also besonders durch einen Übergang von Weichplastik zu Hartplastik. Vorzugsweise sind die Rastlinien jedoch zur Unterstützung der Stülpbarkeit gedacht und stellen z.B. Verdünnungen in der Wandung dar, so dass ein Umfalten oder Stülpen der Wandung entlang der Rastlinien erleichtert wird. Vorzugsweise kann dadurch ein Abschnitt der Wandung bis hin zur nächsten Rastlinie leichter eingestülpt werden, so dass die Rastlinien wie die beweglichen Verbindungsstellen einer Kette bei einem Kettenfahrzeug wirken und immer ein Glied der Kette bis zur nächsten Rastlinie leichter nach innen gestülpt werden kann. Es ist dabei bevorzugt, wenn die Rastlinien geschlossen ringförmig die Wandung umlaufen. Zudem ist bevorzugt, wenn die Rastlinien in gleichem Abstand zueinander verlaufen.

Das Volumen des erfindungsgemäßen Stülpbehälters, aber auch das Volumen von weiteren erfindungsgemäßen Komponentenbehältern liegt bevorzugt zwischen 0,5 und 20 ml, noch bevorzugter zwischen 1 und 20 ml, bevorzugter zwischen 2 und 18 ml, noch bevorzugter zwischen 3 und 16 ml, noch bevorzugter zwischen 4 und 15 ml, noch bevorzugter zwischen 4 und 14 ml, noch bevorzugter zwischen 5 und 13 ml, noch bevorzugter zwischen 5 und 12 ml und noch bevorzugter zwischen 5 und 11 ml. Die Form des Stülpbehälters ist vorzugsweise zylinderförmig, wobei die Wandung bevorzugt rund ist, der Boden vorzugsweise konkav, also nach innen gewölbt ist und die Stirnseite flach ist. Dabei kann der Boden des Stülpbehälters der Form eines Ausdrückstempels einer Vorrichtung zur Aufnahme des Mehrkammermischbehälters angepasst sein. Dieser Ausdrückstempel hat vorzugsweise eine kegelförmige flache Spitzt, welche beispielsweise in eine entsprechende Aussparung des Bodens des Stülpbehälters eingreift oder an einem entsprechend ausgestalteten Boden des Stülpbehälters anliegt. Diese Anpassung der äußeren Form des Bodens oder zumindest eines zentralen Bereichs des Bodens des Stülpbehälters an die Form der Spitze des Ausdrückstempels ist vorteilhaft, weil dadurch ein Abrutschen des Ausdrückstempels vermieden und eine effiziente mechanische Kraftübertragung gewährleistet werden kann. In einer weiteren bevorzugen Ausführungsform ist der Boden des Stülpbehälters außen flach ausgestaltet. Hier drückt sich dann die Spitze des Ausdrückstempels in den Boden ein, so dass dadurch ein Abrutschen des Ausdrückstempels vermieden wird.

Das Material des Bodens des Stülpbehälters weist vorzugsweise eine höhere Steifigkeit auf als das stülpbare, weiche Material der Wandung, ist also bevorzugt aus Hartplastik. Diese Materialverstärkung ist vorteilhaft, damit durch den vom Ausdrückstempel auf den Boden ausgeübten Druck, der Boden nicht beschädigt wird oder sogar einreißt.

Die im Innenraum des Stülpbehälters liegende Oberfläche des Bodens kann eine Ausgestaltung aufweisen, welche das Durchdrücken der Siegelfolien erleichtert. Erfindungsgemäß kann der Boden des Stülpbehälters eine in Richtung der Siegelfolie ausgebildete und mittig angeordnete Durchdrücknase oder Einwölbung oder Verdickung aufweisen oder der Boden kann konkav geformt sein wie z.B. bei einer Sektflasche. Diese Durchdrücknase, Einwölbung, Verdickung oder konkave Ausgestaltung des Bodens kann aus einem härteren Material sein und/oder entsprechend spitz geformt sein, um ein Durchstoßen der Siegelfolien zu erleichtern.

Diese Durchdrücknase oder Bodenausgestaltung kann durch äußeren Druck auf die Stülpseite, besonders also durch Umstülpen der Wandung axial bewegt werden. Die Durchdrücknase oder Bodenausgestaltung dient dazu, die stirnseitige Siegelfolie des Stülpbehälters zu durchstoßen. Weiterhin dient die Durchdrücknase oder Bodenausgestaltung dazu, die Siegelfolie eines angefügten Komponentenbehälters bzw. die Siegelfolien angefügter Komponentenbehälter zu durchstoßen. Sind die Komponentenbehälter vollständig mit Flüssigkeiten gefüllt, so befindet sich die Durchdrücknase vorzugsweise in Nähe der zu durchdrückenden Siegelfolien, damit bereits nach geringem Druckaufwand ein Durchstoßen der Siegelfolien stattfindet, damit sich die Flüssigkeiten mischen können. Befindet sich die Durchdrücknase nicht in unmittelbarer Nähe zu den zu durchstoßenden Siegelfolien, so kann auch die Durchdrücknase derartig flexibel ausgestaltet sein, dass sie sich bei Druck auf die Mitte des Bodens des Stülpbehälters so weit eindrücken lässt, dass die Siegelfolien durchstoßen werden und sich danach flexibel in die Ausgangsposition zurück bewegt und danach erst das Eindrücken des gesamten Bodens zwecks Entleerung des Mehrkammermischbehälters erfolgt. Im Falle dass Komponentenbehälter mit einer Flüssigkeit gefüllt sind ist es bevorzugt, wenn diese vollständig gefüllt sind also ohne Einschluß von Gasen wie Luft oder Inertgasen wie Stickstoff oder Argon. Eine vollständige Füllung einer mit einer Siegelfolie versehenen Komponentenbehälters lässt sich erreichen, indem nach vollständiger Füllung die Siegelfolie z.B. mittels Laser mit dem vollständig gefüllten Komponentenbehälter verschweißt wird, ohne dass dabei ein Eintrag von Gas entsteht. Für den Fall, dass ein Komponentenbehälter mit einem Feststoff oder einer Paste oder einem Gel oder nicht vollständig mit einer Flüssigkeit gefüllt ist, ist eine weitere bevorzugte Ausführungsform, dass die Siegelfolie durchlässig für Gase aber nicht durchlässig für Flüssigkeiten oder Feststoffe ist. In einem solchen Fall kann ein entstehender Druck aufgrund der Komprimierung eines Gases innerhalb eines Komponentenbehälters durch die Siegelfolie hindurch abgebaut werden, indem das Gas durch die Siegelfolie hindurchtritt solange diese noch intakt, d.h. noch nicht durchstoßen oder eingerissen ist. Befindet sich zudem eine Kanüle im Entnahmebereich des Entnahmebehälters, so dann durch die Kanüle das Gas entweichen und damit der Druck in dem Mehrkammermischbehälter abgebaut werden. Für den Fall, dass keine oder noch keine Kanüle zum Abbau des Druckes im Entnahmebereich des Entnahmebehälters eingeführt werden soll, kann auch das Septum, welche den Entnahmebereich des Entnahmebehälters verschießt, durchlässig für Gase aber nicht durchlässig für Flüssigkeiten, Feststoffe oder Zellen sein. In einem solchen Fall kann dann der Druck im Mehrkammermischbehälter durch Entweichung von Gas über das Septum im Entnahmebereich des Entnahmebehälters erreicht werden. Es versteht sich von selbst, dass in einem solchen Fall der Entnahmebereich des Entnahmebehälters nach oben zu halten ist wie bei einer aufgezogenen Spritze, so dass die aufgestiegenen Gase entweichen können. Anstelle eines gasdurchlässigen Septums kann auch ein Ventil verwendet werden.

Eine weitere Variante ist das Durchdrücken der Siegelfolien mit einer durch den Entnahmebereich des Zwischenbehälters eingeführten Kanüle, wobei nach dem Durchstechen die Kanüle zurückgezogen, die Lösungen durchmischt und danach der Mehrkammermischbehälter durch Eindrücken des Bodens und Stülpen des Stülpbehälters entleer wird. Ist in einem Zwischenbehälter oder in dem Entnahmebehälter ein Feststoff eingefüllt, so ist bevorzugt, dass dieser sich unter vermindertem Druck oder unter Vakuum in dem Komponentenbehälter befindet. Bei einer derartigen Ausführungsform genügt zumeist der im Stülpbehälter durch das Eindrücken des Bodens entstehende Druck, um die Siegelfolien zu durchtrennen, bevor der Boden des Stülpbehälters oder die Durchdrücknase die Siegelfolien erreichen. Die Durchdrücknase ist vorzugsweise aus demselben Material wie der Stülpbehälter vor allem dann, wenn der Stülpbehälter mit Rastlinien versehen ist, oder aus einem harten Material, welches den einwirkenden Kräften standhalten kann. Ein hartes Material der Durchdrücknase oder Bodenausgestaltung ist vorteilhaft, weil dadurch eine ausreichende Stabilität zum Durchstoßen einer oder mehrerer Siegelfolien gewährleistet wird. Besonders bevorzugtes Material der Durchdrücknase ist dabei Hartplastik. Die Länge der Durchdrücknase oder Bodenausgestaltung ist dabei mindestens so lang, dass sie beim Einstülpen des Bodens bzw. der Wandung die Siegelfolie der Stirnseite und die Siegelfolie des angrenzenden Entnahmebehälters durchstößt. Weiterhin kann sie so lang sein, dass sie den Stülpbehälter auf seiner gesamten Länge durchzieht. Diese Ausführungsform ist vorteilhaft und darum bevorzugt, weil sie sich besonders zum Durchstoßen mehrerer Versiegelungen eignet, wenn mehrere Komponentenbehälter hintereinander angeordnet sind wie z.B. beim Tri-System oder Quad-System.

Die Durchdrücknase oder die Einwölbung oder die Verdickung oder der konkav geformte Boden der Stülpseite des erfindungsgemäßen Stülpbehälters kann Kanäle, Rillen, Wellen, sternförmige Vertiefungen, Hügel, Noppen, Stifte oder andere Oberflächenunebenheiten aufweisen, die ein schnelles Einströmen oder Überströmen der Flüssigkeit aus dem Stülpbehälter in mindestens einen weiteren Komponentenbehälter ermöglichen. Dabei fließt die Flüssigkeit bevorzugt aus dem Stülpbehälter in den mindestens einen weiteren Komponentenbehälter. Dies ist besonders sinnvoll, wenn sich in dem mindestens einen weiteren Komponentenbehälter ein Feststoff befindet. Die Strömung der Flüssigkeit wird besonders durch die axiale Bewegung der innenliegenden Vorrichtung ermöglicht. Es ist jedoch auch möglich, dass, wenn der Entnahmebehälter eine Flüssigkeit enthält, die Flüssigkeit aus einem der weiteren Komponentenbehälter in Richtung Stülpbehälter fließt und sich die Flüssigkeiten auf diese Weise mischen. Das durch die vorgesehenen Kanäle etc. ermöglichte schnelle Überströmen von Flüssigkeit ist vorteilhaft, um besonders bei einem teilweisen oder vollständigen Einstülpen des Stülpbehälters in den stirnseitig angefügten Komponentenbehälter eine schnelle Durchmischung der Flüssigkeiten oder der Lösung oder Suspension des Feststoffs in der Flüssigkeit zu gewährleisten.

Die runde Form der Wandung ist besonders vorteilhaft und besonders bevorzugt, weil sie die Stülpbarkeit am besten ermöglicht. Jedoch kann die Wandung auch durch Kanten in mehrere Teilflächen eingeteilt sein. Dabei ist bevorzugt, wenn die Seitenwandung in mindestens acht Teilflächen aufgeteilt ist, weil eine geringere Zahl von Teilflächen das Stülpen erschwert. Je kleiner der Innenwinkel zwischen den Kanten ist, desto schwerer wird der Stülpvorgang. Sind die Komponentenbehälter nicht rund sondern eckig ausgestaltet, d.h. ist die Querschnittsfläche nicht kreisförmig sondern eckig, dann sollte der Innenwinkel zwischen den Kanten mindestens 135° betragen. Ein kreisförmiger Querschnitt der Komponentenbehälter und damit eine Zylinderform des Mehrkammermischbehälters sind jedoch am meisten bevorzugt, wobei eine ovale Form auch noch bevorzugt ist.

Die Stirnseite des Stülpbehälters ist die dem Boden gegenüberliegende Seite. Die Stirnseite ist mit einer Siegelfolie verschlossen. Der Stülpbehälter wird mit dem Boden nach unter mit der Flüssigkeit befüllt und danach mit der Siegelfolie verschlossen. Die Siegelfolie ist durchdrückbar, das heißt, dass das Durchdrücken, Durchstoßen bzw. Einreißen der Siegelfolie durch die Wirkung einer Kraft möglich ist. Ist die Siegelfolie des Stülpbehälters und des angrenzenden Komponentenbehälters durchtrennt kann die Flüssigkeit aus dem Stülpbehälter in den angrenzenden Komponentenbehälter fließen.

Die Versiegelung des Stülpbehälters durch eine Siegelfolie dient zum sterilen und stoffschlüssigen Verschluss des Behälters. Die Siegelfolie dient zum Versiegeln des Stülpbehälters bzw. ganz allgemein der Komponentenbehälter, welche unter aseptischen Bedingungen befüllt wurden, so dass der Inhalt eines jeden Komponentenbehälters steril und keimfrei bleibt. Die Siegelfolie zum Verschluss der Komponentenbehälter kann z.B. als Verbundfolie vorliegen. Bevorzugte Materialien sind beispielsweise Metalle wie z.B. Aluminiumfolie, des weiteren Kunststoffe, Metall-Kunststoffverbindungen, Polymere oder Kombinationen davon. In einer bevorzugten Ausführungsform ist die Versiegelung als "Weak Seal" ausgeführt, d.h. an einer oder mehrerer Stellen ist das Material der Siegelfolie besonders schwach, so dass es sich leichter durchdrücken lässt, oder es sind Vorstanzungen eingefügt, so dass das Material an diesen Stellen leichter einreißt. Wichtig ist jedoch, dass die Siegelfolie die Vorrichtung vollständig und luftdicht abschließt, um eine sterile Aufbewahrung der Inhalte zu gewährleisten und auch bei gekühlter Lagerung des Mehrkammermischbehälters aufgrund der Materialkontraktion bei Kälte bis vorzugsweise 0°C, weiter bevorzugt -15°C und insbesondere bevorzugt -25°C nicht einreißt. Einem Fachmann sind ausreichend Materialien und Möglichkeiten bekannt, eine solche Siegelfolie zu realisieren. Bevorzugte Materialien sind beispielsweise Metalle wie z.B. Aluminiumfolie, des weiteren Kunststoffe, Metall-Kunststoffverbindungen, Polymere oder Kombinationen davon.

In einer bevorzugten Ausführungsform hat der Stülpbehälter eine Nut an der Außenseite der Wandung oder Einbuchtung in der Außenseite der Wandung. Diese Nut oder Einbuchtung ist vorteilhaft, weil sie in einer Vorrichtung zur Aufnahme des Stülpbehälters, die ein Gegenstück dazu, also eine entsprechende Ausbuchtung oder einen Aufsatz oder einen Zapfen hat, ein Drehen des eingesetzten Stülpbehälters verhindert und eine stabile Position garantiert. Diese Nut oder Einbuchtung befindet sich bevorzugt in Höhe der Stirnseite oder entlang der Kontaktfläche oder Kontaktlinien zweier Komponentenbehälter. Diese Ausführung ist vorteilhaft, um das Einstülpen der Wandung nicht zu behindern. Genauso kann es aber erwünscht sein, dass die Nut oder Einbuchtung das Einstülpen an einer gewissen Stelle begrenzt oder stoppt. Die Anordnung der Nut oder Einbuchtung an einer anderen Höhe der Wandung ist daher ebenfalls bevorzugt. Die Form der Nut oder Einbuchtung ist dabei länglich, und bevorzugt rund, jedoch sind kantige Formen auch möglich. Es ist auch möglich und bevorzugt, wenn der Stülpbehälter statt der Nut oder Einbuchtung eine Ausbuchtung oder einen Aufsatz oder einen Zapfen hat und die besagte Vorrichtung zur Aufnahme des Stülpbehälters eine entsprechende Ausformung aufweist. Diese erfindungsgemäße Vorrichtung zur Aufnahme des Mehrkammermischbehälters wird weiter unten noch ausführlicher beschrieben und umfassend eine halbschalenförmige Aufnahme für den Mehrkammermischbehälter, Haltemittel für den Mehrkammermischbehälter und einen in axialer Richtung beweglichen Ausdrückstempel.

Der erfindungsgemäße Stülpbehälter weist weiterhin bevorzugt einen Fügerand auf. Dieser Fügerand ist vorteilhaft, weil er ein rutschfestes Zusammenfügen mit anderen Behältern ermöglicht und dem Zusammenschluss einen mechanischen Halt vermittelt. Der Fügerand ist dabei an der Stirnseite des Stülpbehälters angebracht. Dabei besteht der Fügerand idealerweise aus demselben Material wie der Stülpbehälter, und zwar wie der nichtstülpbare Teil des Stülpbehälters falls ein solcher Teil vorhanden ist. Zum effizienteren Zusammenfügen von zwei Behältern ist es vorteilhaft, wenn der Fügerand eines Behälters eine Nutung und der andere eine dazu passende Spundung aufweist. Möglich ist auch, dass die Fügeränder einander entsprechende Schraubmöglichkeiten aufweisen. Der Fügerand kann weiterhin bevorzugt auf der Außenseite die besagte Nut, Einbuchtung, Ausbuchtung oder Zapfen aufweisen, die ein Drehen oder Rutschen in einer Vorrichtung zur Aufnahme des Stülpbehälters verhindern soll.

Der erfindungsgemäße Entnahmebehälter wird in seiner äußeren Form durch Stirnseite (Oberseite), Wandung und Unterseite charakterisiert. Die Oberseite des Entnahmebehälters ist wie die Stirnseite (Oberseite) beim Stülpbehälter durch eine durchdrückbare Folie versiegelt, so dass der Inhalt des Entnahmebehälters steril bleibt. Für das Material und die Gestaltung der Siegelfolie gelten dabei dieselben bevorzugten Kriterien wie für die Siegelfolie des Stülpbehälters. Der Entnahmebehälter kann dabei eine Flüssigkeit, eine Lösung oder einen festen Stoff oder ein Feststoffgemisch enthalten. Feste Stoffe sind bevorzugt pulverförmig, weiter bevorzugt lyophilisiert oder gefriergetrocknet. Die Form des Entnahmebehälters ist dabei vorzugsweise der des Stülpbehälters angepasst, d.h. der Durchmesser und die Form der Unterseite entspricht der des Stülpbehälters, wodurch eine dichte, also stoffschlüssige Verbindung gewährleistet werden kann. Es ist also wichtig, dass Stirnseite des Stülpbehälters und Unterseite des Entnahmebehälters beim Bi-System (also ohne Zwischenbehälter) gut zusammenpassen, d.h. bevorzugt denselben Durchmesser haben sollten und vorzugsweise auch dieselbe Wandstärke aufweisen sollten.

Das Material des Entnahmebehälters weist vorzugsweise eine steife Beschaffenheit auf, und ist dabei vorzugsweise so beschaffen wie der nichtstülpbare Teil des Stülpbehälters, falls ein solcher Teil vorhanden ist. Es ist nicht erforderlich, dass der Entnahmebehälter stülpbar ist, vielmehr sollte der Entnahmebehälter nicht stülpbar sein und die Wandung des Entnahmebehälters sollte dem Druck ausgeübt durch den Ausdrückstempel standhalten und nicht deformiert werden. Der Entnahmebehälter ist also vorzugsweise aus Metall oder einem Polymer, bevorzugter aus Plastik, und besonders bevorzugt aus Hartplastik.

Weiterhin ist es vorteilhaft, wenn auch die Wandung des Entnahmebehälters die gleiche Form aufweist wie die des Stülpbehälters, so dass beide Behälter in dieselbe Vorrichtung zur Aufnahme des Mehrkammermischbehälters passen. Weiterhin ist die gleiche Form vorteilhaft, weil dadurch der Stülpbehälter ohne Lücken oder mechanische Behinderung in den Entnahmebehälter gestülpt werden kann (Figur 4). Es ist darum weiterhin vorteilhaft, wenn der Entnahmebehälter der spiegelverkehrten Form des Stülpbehälters entspricht. Der Entnahmebehälter weist also bevorzugt das gleiche Volumen und die gleiche Länge auf wie der Stülpbehälter. Es ist jedoch auch möglich, dass der Entnahmebehälter größere oder kleinere Volumina und Längen aufweist als der Stülpbehälter, wobei der Durchmesser beider Komponentenbehälter gleich oder maximal um 10% verschieden voneinander sein sollte.

Der Entnahmebehälter weist ebenfalls wie der Stülpbehälter vorzugsweise eine Nut, Einbuchtung, Ausbuchtung oder einen Zapfen aufweisen, die ein Drehen oder Verrutschen in einer Vorrichtung zur Aufnahme des Mehrkammermischbehälters verhindern soll. Dabei ist es besonders bevorzugt, wenn die Nuten, Einbuchtungen, Ausbuchtungen oder Zapfen bei den zusammengefügten Behältern entlang der Verbindungsnaht liegen.

Der Entnahmebehälter weist zudem einen Entnahmebereich auf und besitzt bevorzugt einen von einer Nadel durchstechbaren Bereich auf der Unterseite des Entnahmebehälters. Der Entnahmebereich verschließt den Entnahmebehälter hermetisch, so dass der Inhalt steril und keimfrei bleibt und dennoch durch den Entnahmebereich die frisch hergestellte Lösung entnommen werden kann. Bevorzugt ist ein von einer Nadel durchstechbaren Bereich wie z.B. ein Stopfen oder ein Septum, so dass mittels einer Nadel oder einer Kanüle die Lösung entnommen und in eine Spritze aufgezogen oder in einen Behälter oder Beutel überführt werden kann. Der Stopfen kann vorteilhafter Weise durch eine Metallbördelkappe, also ein äußerer Verschluss vorzugsweise aus Aluminiumblech, mit dem Behälter formschlüssig vercrimpt werden. Möglich für Verschlußsysteme dieser Art sind sogenannte Tip-Cap-Verschlüsse. Für einen optimalen Formschluss in Verbindung mit dem Crimpen ist der über den Stopfen hinausgehende Bereich idealerweise mit einem trichterförmigen Hinterschnitt versehen, also einen Rand, um den herum die Bördelkappe vercrimpt werden kann. Prinzipiell besteht auch die Möglichkeit, den Stopfen von der Innenseite des Behälters einzulegen und zu verschweißen, wodurch die Bördelkappe entfallen könnte. Um die Kontaktfläche des Inhalts mit dem Stopfen oder Septum so gering wie möglich zu halten, ist die Variante mit der Bördelkappe vorteilhafter und darum bevorzugt.

Weiterhin ist ein nadelfreier Zugang zum Entnahmebehälter möglich und bevorzugt. Bei einem nadelfreien Zugang wird ein Polymerschlauch mit einem Membranventil durch den Stopfen in den Entnahmebehälter gelegt. Vorstellbar sind dabei nadelfreie Zugänge, wie man sie aus der Infusionstherapie kennt, z.B. beim sogenannten Tropf. Der nadelfreie Zugang ermöglicht eine sichere und bequeme Entnahme des gemischten Inhalts der verbundenen Behälter aus dem Entnahmebehälter. Der Inhalt wird dabei in einen Auffangbehälter wie z.B. einem Tropfbeutel geleitet. Dabei bildet vorzugsweise der Entnahmebehälter mit dem nadelfreien Zugang und dem Auffangbehälter ein geschlossenes, steriles System. Zudem ist es bevorzugt, wenn der Entnahmebehälter ein druckgeregeltes Ausströmventil oder Überdruckventil zum Entweichen von überschüssiger Luft aufweist.

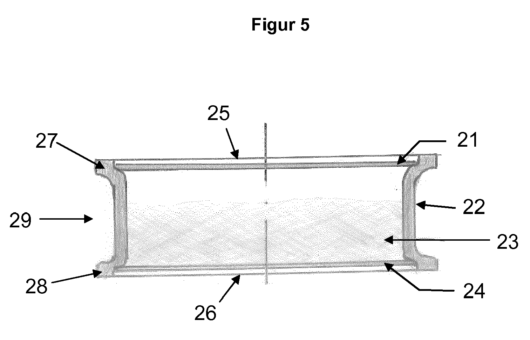

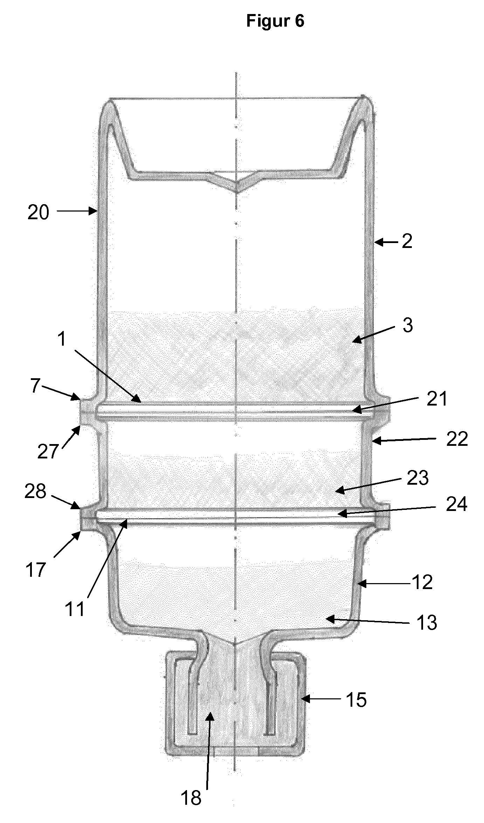

In einer weiteren bevorzugten Ausführungsform besteht der erfindungsgemäße Mehrkammermischbehälter aus einem Stülpbehälter und einem Entnahmebehälter und einem zwischen Stülpbehälter und Entnahmebehälter befindlichen Zwischenbehälter (Figur 5) gefüllt mit einer Flüssigkeit oder einem Feststoff, wobei der Zwischenbehälter an der Oberseite und an der Unterseite mit einer durchdrückbaren Siegelfolie verschlossen ist und der Zwischenbehälter dichtend mit dem Stülpbehälter und dichtend mit dem Entnahmebehälter verbunden ist. In dieser Ausführungsform ist der Mehrkammermischbehälter ein Tri-System (Figur 6).

Der Zwischenbehälter ist also ein weiterer Komponentenbehälter, der eine dritte Komponente zum Mischen bereitstellt. Der Zwischenbehälter wird durch eine Wandung und eine Oberseite als auch eine Unterseite charakterisiert. Sowohl Oberseite als auch Unterseite sind jeweils mit einer durchdrückbaren Siegelfolie verschlossen. Für das Material der Siegelfolie gelten dabei die bevorzugten Kriterien, die bei Stülp- und Entnahmebehälter erwähnt wurden. In dem Mehrkammermischbehälter müssen also insgesamt vier Siegelfolien durchstoßen werden (Stirnseite des Stülpbehälters, Ober- und Unterseite des Zwischenbehälters, Unterseite des Entnahmebehälters), um Kontakt und Durchmischung aller Komponenten zu gewährleisten.

Die Form des Zwischenbehälters ist dabei vorzugsweise der der anderen Komponentenbehälter angepasst, d.h. entspricht ihnen in Durchmesser und Form, wodurch eine dichte, also stoffschlüssige Verbindung mit den anderen Komponentenbehältern gewährleistet werden kann.

Das Volumen des Zwischenbehälters kann dabei eine beliebige Größe haben, bevorzugt jedoch dem der anderen Komponentenbehälter entsprechen. Bevorzugter ist das Volumen des Zwischenbehälters jedoch kleiner als das von Stülpbehälter oder Entnahmebehälter. Grundsätzlich gibt es jedoch keine Limitierung im Volumen der Komponentenbehälter. Da alle Komponentenbehälter weitgehend denselben Durchmesser aufweisen sollten, wird somit das Volumen des jeweiligen Komponentenbehälters über seine Höhe bestimmt. Da ferner bevorzugt ist, dass nur der Stülpbehälter stülpbar ausgestaltet ist und Entnahmebehälter als auch Zwischenbehälter nicht stülpbar sondern weitgehend formstabil sind, sollte die Höhe des Stülpbehälters ungefähr der Gesamthöhe von Entnahmebehälter und allen Zwischenbehältern entsprechen, damit der Stülpbehälter vollständig in den oder die Zwischenbehälter und den Entnahmebehälter gestülpt werden kann.

Das Material der Wandung des Zwischenbehälters weist vorzugsweise eine steife Beschaffenheit auf, und ist dabei vorzugsweise so beschaffen wie der Entnahmebehälter. Die Wandung des Zwischenbehälters ist also vorzugsweise aus Metall oder einem Polymer, bevorzugter aus Plastik, und besonders bevorzugt aus Hartplastik.

In einer weiteren bevorzugten Ausführungsform kann die Wandung des Zwischenbehälters jedoch auch aus Weichplastik bestehen, also besonders biegsamem Kunststoff wie beispielsweise Weich-PVC oder Polyolefine, z.B. Polyethylen. Durch dieses Material wird eine Stülpbarkeit erreicht. Diese Stülpbarkeit ist vorteilhaft, weil sie ein über das Einstülpen des Stülpbehälters hinausgehendes Stülpen ermöglicht. Das kann z.B. notwendig sein, wenn der Zwischenbehälter zu groß ist, um ein Durchdrücken der unteren Siegelfolie des Zwischenbehälters und des angefügten Entnahmebehälters durch den Durchdrückstempel des Stülpbehälters zu ermöglichen. Der Zwischenbehälter sollte daher immer dann zumindest teilweise oder auch vollständig stülpbar sein, wenn die Höhe des Entnahmebehälters plus die Höhe des Zwischenbehälters größer ist als die Höhe des Stülpbehälters. Ist die Höhe des Entnahmebehälter so groß wie die Höhe des Zwischenbehälters plus die Höhe des Stülpbehälters, dann sollte der Zwischenbehälter auch über seine gesamte Höhe stülpbar sein, damit Zwischenbehälter und Stülpbehälter vollständig in den Entnahmebehälter gestülpt werden können.

Eine bevorzugte Ausführungsform der vorliegenden Erfindung betrifft daher einen Mehrkammermischbehälter bestehend aus einem Stülpbehälter und einem Entnahmebehälter und einem zwischen Stülpbehälter und Entnahmebehälter befindlichen Zwischenbehälter gefüllt mit einer Flüssigkeit oder einem Feststoff, wobei der Zwischenbehälter an der Oberseite und an der Unterseite mit einer durchdrückbaren Siegelfolie verschlossen ist und der Zwischenbehälter dichtend mit dem Stülpbehälter und dichtend mit dem Entnahmebehälter verbunden ist.

Vorzugsweise weisen bei dem Mehrkammermischbehälter zur dichtenden Verbindung zwischen Stülpbehälter und Zwischenbehälter und zwischen Zwischenbehälter und Entnahmebehälter, der Stülpbehälter, der Zwischenbehälter und der Entnahmebehälter in Höhe der Siegelfolien eine Nut, eine Wulst, ein Gewinde oder eine Einbuchtung auf.

Erfindungsgemäß ist die äußere Form der Komponentenbehälter zylinderförmig mit übereinstimmenden oder weitgehend übereinstimmenden Außenradien. Bei diesem Mehrkammermischbehälter aus drei Komponentenbehältern befindet sich im Stülpbehälter eine Flüssigkeit und im Zwischenbehälter eine Flüssigkeit oder ein Feststoff als auch im Entnahmebehälter eine Flüssigkeit oder ein Feststoff. Ein derartiger Mehrkammermischbehälter kann aber auch vom Arzt oder Klinikpersonal aus den einzelnen Komponentenbehältern eines hierin offenbarten Kits selbst zusammengesetzt werden, wodurch eine große Flexibilität hinsichtlich der Wahl und Menge des Lösungsmittels sowie der Wahl, Menge und Konzentration der Wirkstoffe besteht.

In einer weiteren bevorzugten Ausführungsform besteht der erfindungsgemäße Mehrkammermischbehälter aus dem Stülpbehälter und dem Entnahmebehälter und zwei zwischen Stülpbehälter und Entnahmebehälter befindlichen Zwischenbehältern jeweils gefüllt mit einer Flüssigkeit oder einem Feststoff, wobei beide Zwischenbehälter an der Oberseite und an der Unterseite mit einer durchdrückbaren Siegelfolie verschlossen sind und beide Zwischenbehälter dichtend miteinander verbunden sind und der eine Zwischenbehälter dichtend mit dem Stülpbehälter und der andere Zwischenbehälter dichtend mit dem Entnahmebehälter verbunden ist. In dieser Ausführungsform ist der Mehrkammermischbehälter ein Quad-System.

Somit ist die vorliegende Erfindung auch auf Mehrkammermischbehälter gerichtet, die aus einem Stülpbehälter und einem Entnahmebehälter und zwei zwischen Stülpbehälter und Entnahmebehälter befindlichen Zwischenbehältern jeweils gefüllt mit einer Flüssigkeit oder einem Feststoff bestehen, wobei beide Zwischenbehälter an der Oberseite und an der Unterseite mit einer durchdrückbaren Siegelfolie verschlossen sind und beide Zwischenbehälter dichtend miteinander verbunden sind und der eine Zwischenbehälter dichtend mit dem Stülpbehälter und der andere Zwischenbehälter dichtend mit dem Entnahmebehälter verbunden ist.

Dabei sind Mehrkammermischbehälter bevorzugt, wobei zur dichtenden Verbindung zwischen Stülpbehälter und dem einen Zwischenbehälter und zwischen dem einen und dem anderen Zwischenbehälter und zwischen dem anderen Zwischenbehälter und dem Entnahmebehälter, der Stülpbehälter, der eine Zwischenbehälter, der andere Zwischenbehälter und der Entnahmebehälter in Höhe der Siegelfolien eine Nut, eine Wulst, ein Gewinde oder eine Einbuchtung aufweisen.

Bevorzugt sind ferner Mehrkammermischbehälter, wobei der Mehrkammermischbehälter bestehend aus Stülpbehälter und Entnahmebehälter außen entlang der Verbindungslinie zwischen Stülpbehälter und Entnahmebehälter eine Nut aufweist oder der Mehrkammermischbehälter bestehend aus Stülpbehälter, Zwischenbehälter und Entnahmebehälter außen entlang der Verbindungslinie zwischen Stülpbehälter und Zwischenbehälter und/oder zwischen Zwischenbehälter und Entnahmebehälter eine Nut aufweist oder der Mehrkammermischbehälter bestehend aus Stülpbehälter, zwei Zwischenbehältern und Entnahmebehälter außen entlang der Verbindungslinie zwischen Stülpbehälter und dem einen Zwischenbehälter und/oder zwischen den beiden Zwischenbehältern und/oder zwischen dem anderen Zwischenbehälter und Entnahmebehälter eine Nut aufweist. Diese Nut dient zum einen zur dichtenden Verbindung der Komponentenbehälter miteinander als auch zur Fixierung des Mehrkammermischbehälters in der Vorrichtung zur Aufnahme des Mehrkammermischbehälter. Eine andere bevorzugte Weise zur dichtenden Verbindung der Komponentenbehälter miteinander ist über ein Gewinde, wobei ein glatter Außenmantel ohne Nuten entsteht, also ein glatter Zylinder, der in eine entsprechende rohrförmige Vorrichtung eingeführt werden kann, um den Mehrkammermischbehälter darin wie üblich mittels Druck auf den Boden des Stülpbehälters zu entleeren.

Der zweite Zwischenbehälter ist also ein weiterer Komponentenbehälter zusätzlich zu dem oben beschriebenen (ersten) Zwischenbehälter, der eine vierte Komponente zum Mischen bereitstellt. Der zweite Zwischenbehälter wird durch eine Wandung und zwei durch eine Siegelfolie versiegelte Seiten charakterisiert. Für das Material der Siegelfolie gelten dabei die bevorzugten Kriterien, die bei Stülp-, Entnahme- und erstem Zwischenbehälter erwähnt wurden. In dem Mehrkammermischbehälter müssen also insgesamt sechs Siegelfolien durchstoßen werden (Stirnseite des Stülpbehälters, Ober- und Unterseite des ersten Zwischenbehälters, Ober- und Unterseite des zweiten Zwischenbehälters, Oberseite des Entnahmebehälters), um Kontakt und Durchmischung aller Komponenten zu ermöglichen.

Es ist jedoch auch möglich und bevorzugt, dass die vier Komponentenbehälter so miteinander verbunden sind, dass sich jeweils nur eine Siegelfolie zwischen den entsprechenden Komponenten befindet. Dann würden in dem Mehrkammermischbehälter nur drei Siegelfolien durchstoßen werden müssen, um Kontakt und Durchmischung der Komponenten zu gewährleisten.

Die Form des zweiten Zwischenbehälters ist dabei vorzugsweise der der anderen Komponentenbehälter angepasst, d.h. entspricht ihnen in Durchmesser und Form, wodurch eine dichte, also stoffschlüssige Verbindung gewährleistet werden kann.

Für das Volumen und die Größe des zweiten Zwischenbehälters gelten dabei die gleichen Kriterien wie für den ersten Zwischenbehälter. Dabei können die Zwischenbehälter dieselbe Größe aufweisen, aber auch unterschiedliche groß sein. Das gleiche gilt für das Material der Wandung des zweiten Zwischenbehälters. Auch der zweite Zwischenbehälter kann stülpbar sein ist es jedoch vorzugsweise nicht. Es ist jedoch besonders bevorzugt, wenn von zwei Zwischenbehältern nur einer stülpbar ist. Ist ein Zwischenbehälter stülpbar, so ist dies der mit dem Stülpbehälter verbundene Zwischenbehälter.

In weiteren möglichen bevorzugten Ausführungsformen können auch mehr als zwei Zwischenbehälter in dem Mehrkammermischsystem enthalten sein. In diesem Falle würde man von einem Multi-System sprechen. Dabei ist es bevorzugt, wenn mindestens ein Zwischenbehälter stülpbar ist.

Der oder die Zwischenbehälter weisen wie Entnahmebehälter und Stülpbehälter vorzugsweise eine Nut, Einbuchtung, Ausbuchtung oder einen Zapfen auf, die ein Drehen oder Verrutschen in einer Vorrichtung zur Aufnahme des Mehrkammermischbehälters verhindern soll. Dabei ist es besonders bevorzugt, wenn sich die Nuten, Einbuchtungen, Ausbuchtungen oder Zapfen bei den zusammengefügten Komponentenbehältern entlang der Kontaktflächen der Komponentenbehälter befinden.

Der erfindungsgemäße Mehrkammermischbehälter weist also mehrere Nuten, Einbuchtungen, Ausbuchtungen oder Zapfen auf, die in einer Linie liegen können und mit entsprechenden Zapfen, Ausbuchtungen, Einbuchtungen oder Nuten einer Vorrichtung zur Aufnahme des Mehrkammermischbehälters zusammenpassen, wodurch der Mehrkammermischbehälter in der Vorrichtung rutsch- bzw. drehfest gelagert werden kann.

Alle Zwischenbehälter können bevorzugt einen Fügerand aufweisen. Alle Zwischenbehälter weisen weiterhin vorzugsweise an der Oberseite und/oder der Unterseite, also in der Verbindungsebene der Komponentenbehälter eine Nut oder Einbuchtung oder auch einen Zapfen oder eine Ausbuchtung auf.

Zur Erfindung gehört weiterhin eine Vorrichtung zur Aufnahme des Mehrkammermischbehälters, um die Stülpung durchzuführen und dadurch die gewünschte Lösung frisch zuzubereiten. Diese Vorrichtung umfassend eine halbschalenförmige Aufnahme für den Mehrkammermischbehälter, Haltemittel für den Mehrkammermischbehälter und einen in axialer Richtung beweglichen Ausdrückstempel. Die Vorrichtung ist vorzugsweise ein karpulenartiges System, das zur Durchmischung der Inhaltsstoffe der Komponentenbehälter verwendet wird (Figur 4). Die Vorrichtung ist als halbschalenförmige Aufnahme mit einer oder mehreren Zentriernuten, Einbuchtungen, Zapfen oder Ausbuchtungen als Haltemittel in der Verbindungsebene der Behälter ausgeführt, die den Mehrkammermischbehälter zentriert und in seiner Lage hält. Die Vorrichtung ist dadurch charakterisiert, dass sie den Mehrkammermischbehälter aufnehmen kann, in seiner Lage durch die Haltemittel stabilisiert und weiterhin einen in axialer Richtung beweglichen Ausdrückstempel zur Applikation von äußerem Druck auf den Boden des Stülpbehälters aufweist.

Der in axiale Richtung bewegliche Ausdrückstempel hat vorzugsweise eine Zentrierspitze, die in die vorgesehene Aussparung im Boden des Stülpbehälters passt. Die Zentrierspitze ist vorteilhaft, weil sie eine effiziente Kraftübertragung von Ausdrückstempel auf den Boden des Stülpbehälters ermöglicht und ein Abrutschen des Ausdrückstempels verhindert. Der Ausdrückstempel hat weiterhin vorzugsweise einen Stoppring, um ein unerwünscht weites Eindrücken in die Vorrichtung zu begrenzen.
Die Vorrichtung für ein Tri-, Quad- oder Multi-System ist prinzipiell ähnlich aufgebaut, lediglich Länge der Vorrichtung und Anzahl der Zentriernuten sind an die jeweilige Konfiguration des Mehrkammermischbehälters angepasst.

Zur Erfindung gehört weiterhin eine Methode zur aseptischen Herstellung einer Lösung aus mindestens zwei Komponenten, charakterisiert durch das Einlegen eines Mehrkammermischbehälters in die Vorrichtung zur Aufnahme des Mehrkammermischbehälters, Ausübung von Druck durch den Ausdrückstempel auf den Boden des Stülpbehälters, Drücken des Bodens des Stülpbehälters in Richtung des Entnahmebehälters, wobei sich die Wandung des Stülpbehälters kontinuierlich nach innen stülpt und mit forschreitendem Drücken die Siegelfolien durchdrückt werden und Vermischung der mindestens zwei Komponenten.

Nach der Herstellung der Lösung unter aseptischen Bedingungen erfolgt die Entnahme der hergestellten Lösung durch den Entnahmebereich des Entnahmebehälters mittels Nadel oder auch nadelfrei wie oben geschildert.

Mischbare Komponenten für bevorzugte parenterale Anwendungen, welche in dem Mehrkammermischbehälter vorzugsweise als Feststoff gelagert und kurz vor der Anwendung mit dem im Mehrkammermischbehälter getrennt gelagerten Lösungsmittel vermischt werden können, sind beispielsweise Antibiotika, Analgetika, anti-entzündliche Wirkstoffe, Steroide, antiproliferativen, immunsuppressive, fungizide, zytostatische, antimigrative, antiphlogistische, zytotoxische, antiangiogene oder/und antithrombotische Wirkstoffe, Vitamine, Carotinoide, Schmerzmittel, oder Aminosäuren, oder andere Wirkstoffe. Flüssigkeiten zur Lösung oder Mischung umfassen vorzugsweise aqua bidest., isotonische Natriumchloridlösung, oder verschiedene andere isotonische Salzlösungen.

Im Folgenden wird die vorliegende Erfindung an zwei Beispielen verdeutlicht, welche konkrete Ausführungsformen offenbaren jedoch nicht beschränkend für den Schutzumfang der Erfindung sein sollen. Für einen Fachmann naheliegende Abwandlungen und Änderungen der Erfindung fallen dementsprechend unter den Schutzumfang der Patentansprüche.

### Referenzzeichen

- 1: Siegelfolie
- 2: Wandung des Stülpbehälters
- 3: Flüssigkeit
- 4: Durchdrücknase oder Einwölbung oder Verdickung
- 5: Stülpbehälter
- 6: Ausformung zur Aufnahme des Ausdrückstempels
- 7: Nut oder Wulst
- 8: Boden des Stülpbehälters
- 9: Stirnseite oder Oberseite des Stülpbehälters
- 10: Rastlinien
- 11: Siegelfolie
- 12: Wandung des Entnahmebehälters
- 13: Feststoff oder Flüssigkeit
- 14: Vertiefung
- 15: Verschluß (Stopfen, Septum, Ventil)
- 16: Entnahmebehälter
- 17: Nut oder Wulst
- 18: Entnahmebereich
- 19: Stirnseite oder Oberseite
- 20: Mehrkammermischbehälter
- 21: Siegelfolie
- 22: Wandung des Zwischenbehälters
- 23: Feststoff oder Flüssigkeit
- 24: Siegelfolie
- 25: Oberseite
- 26: Unterseite
- 27: Nut oder Wulst
- 28: Nut oder Wulst
- 29: Zwischenbehälter
- 30: Vorrichtung zur Aufnahme des Mehrkammermischbehälters
- 31: Griffplatte
- 32: Ausdrückstempel
- 33: Verdickung oder Ring
- 34: Zentrierspitze
- 35: Haltemittel
- 36: Kanüle oder Nadel

### Figurenbeschreibung

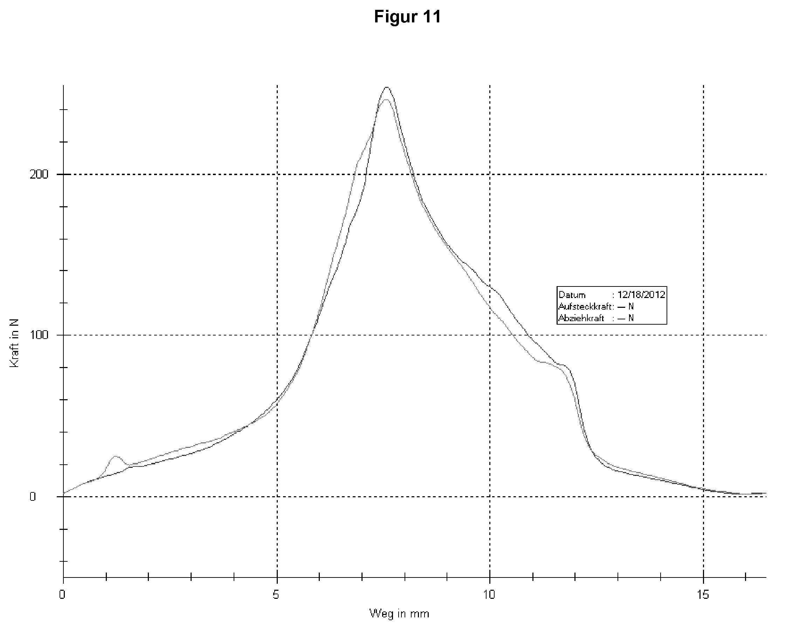

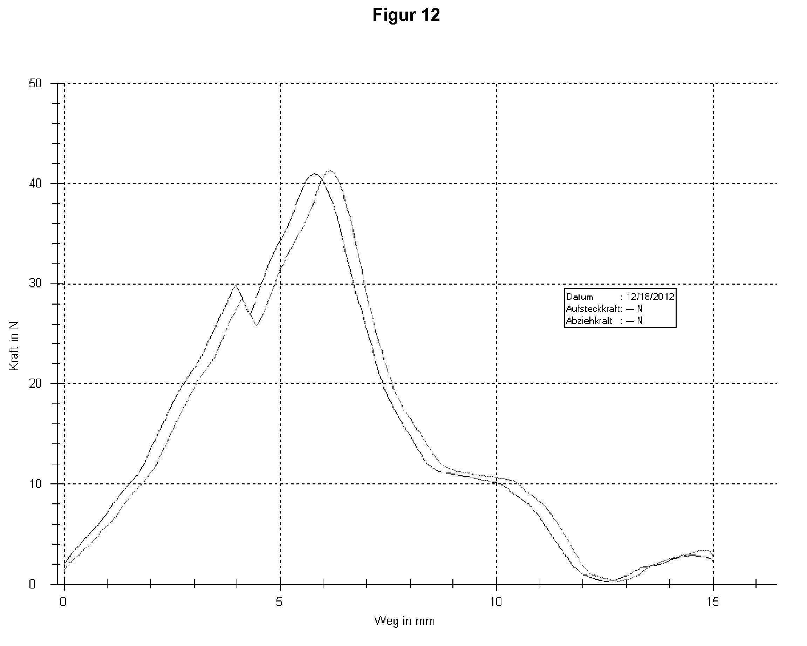

**Figur 1**
   Figur 1 zeigt als Komponentenbehälter einen Stülpbehälter (5), der ca. bis zur Hälfte mit einer Flüssigkeit (3) gefüllt ist. Die Stirnseite oder Oberseite (9) des Stülpbehälters (5) ist mit einer Siegelfolie (1) verschlossen, so dass der Inhalt des Stülpbehälters (5) aseptisch, steril und keimfrei beleibt. Entlang des Randes der Oberseite (9) des Stülpbehälters (5) verläuft eine Wulst oder Nut (7), welche zum dichtenden und/oder stoffschlüssigen Zusammenfügen der Komponentenbehälter vorteilhaft ist und auch als Mittel zum Festhalten in der Vorrichtung (30) zur Aufnahme des Mehrkammermischbehälters (20) dient. Die Siegelfolie (1) ist derart ausgestaltet, dass sie durch die Durchdrücknase (4) oder Einwölbung (4) oder Verdickung (4) durchstoßen werden kann. Mittig auf dem Boden (8) des Stülpbehälters (5) befindet sich die Durchdrücknase (4) oder Einwölbung (4) oder Verdickung (4). Zudem ist der Boden (8) des Stülpbehälters (5) zylinderförmig nach innen ausgeformt, so dass sich eine Aussparung (6) oder Ausformung (6) zur Aufnahme des Ausdrückstempels (32) ergibt. Der Stülpbehälter (5) weist zudem eine umlaufende Wandung (2) auf, welche aus einem elastischen Polymer besteht. Die Wandung (2) ist zudem mit umlaufenden Rastlinien (10) versehen (nicht gezeigt), so dass ein Stülpen der Wandung (2) von einer bis zur nächsten Rastlinie (10) erleichtert wird. Die Wandung (2) des Stülpbehälters (5) ist so ausgestaltet, dass durch Ausübung von Druck auf den Boden (8) des Stülpbehälters (5) in Richtung der Siegelfolie (1) bzw. in Richtung der Oberseite (9) die Wandung (2) sich am Boden (8) des Stülpbehälters (5) umstülpt und nach innen gedrückt wird, d.h. in die Aussparung oder Ausformung (6), so dass kontinuierlich der Boden (8) des Stülpbehälters (5) in Richtung Siegelfolie (1) wandert. Erreicht der Boden (8) des Stülpbehälters (5) die Siegelfolie (1), dann ist der Stülpbehälter (5) nur noch halb so hoch wie am Anfang, da sich die Hälfte der Außenwandung nun im Inneren befindet.
Figur 2
   Figur 2 zeigt als Komponentenbehälter einen Entnahmebehälter (16), der ca. zu einem Drittel mit einem Feststoff (13) oder einer Flüssigkeit (13) gefüllt ist. Die Oberseite (19) des Entnahmebehälter (16) ist mit einer Siegelfolie (11) verschlossen, so dass der Inhalt des Entnahmebehälter (16) aseptisch, steril und keimfrei beleibt. Die Siegelfolie (11) ist so ausgestaltet, dass sie durch den auf den Boden (8) des Stülpbehälters (5) ausgeübten Druck durchstoßen werden kann. Entlang des Randes der Oberseite (19) des Entnahmebehälters (16) verläuft eine Wulst oder Nut (17), welche zum dichtenden und/oder stoffschlüssigen Zusammenfügen der Komponentenbehälter vorteilhaft ist und auch als Mittel zum Festhalten in der Vorrichtung (30) zur Aufnahme des Mehrkammermischbehälters (20) dient. Die Siegelfolie (11) ist derart ausgestaltet, dass sie durch die Durchdrücknase (4) oder Einwölbung (4) oder Verdickung (4) des Stülpbehälters (5) durchstoßen werden kann. An der Unterseite weist der Entnahmebehälter (16) einen Entnahmebereich (18) auf, durch den die frisch hergestellte Lösung aus der Komponente des Stülpbehälters (5) und der Komponente des Entnahmebehälters (16) mittels einer Nadel oder Kanüle oder auch nadelfrei entnommen werden kann. Der Entnahmebereich (18) befindet sich in dem Verschluss (15) oder bildet einen Teil des Verschlusses (15). Der Verschluss (15) kann ein Stopfen oder ein Septum sein, welches beispielsweise mittels einer Bördelkappe um den Hals der Unterseite des Entnahmebehälters (16) befestigt ist. Der Stopfen oder das Septum kann aber auch mit dem Material des Entnahmebehälters (16) verklebt oder verschweißt werden. In diesem Fall kann die Bördelkappe entfallen. Der Stopfen oder das Septum können mit einer Nadel oder Kanüle durchstochen werden, so dass die frisch hergestellte Lösung aus dem Mehrkammermischbehälter (20) entnommen werden kann. Unterhalb des Stopfens oder unterhalb des Septums im Inneren des Entnahmebehälters (16) befindet sich der Entnahmebereich (18), nämlich dort, wo die Spitze der Nadel oder der Kanüle endet. Am Boden des Entnahmebehälters (16) kann sich auch eine Vertiefung (14) oder Aussparung (14) befinden. Diese Vertiefung (14) oder Aussparung (14) dient zur Aufnahme der Durchdrücknase (4) oder Einwölbung (4) oder Verdickung (4) des Stülpbehälters (5). Die Lösung kann auch nadelfrei aus dem Mehrkammermischbehälter (20) entnommen werden, wenn der Verschluss (15) als Ventil ausgestaltet ist. Der Entnahmebehälter (16) weist zudem eine umlaufende Wandung (12) auf, welche aus einem festen oder starren Polymer besteht. Der Entnahmebehälter (16) ist nicht stülpbar ausgestaltet. Daher besteht der Entnahmebehälter (16) aus einem festen Material, welches dem Druck auf den Boden (8) des Stülpbehälters (5) standhalten kann, ohne deformiert zu werden.
Figur 3
   Figur 3 zeigt einen Mehrkammermischbehälter (20) bestehend aus einem Stülpbehälter (5) und einem Entnahmebehälter (16), welche dichtend miteinander verbunden sind, eingelegt in eine Vorrichtung (30) zur Aufnahme des Mehrkammermischbehälters (20). Die Vorrichtung (30) umfasst eine halbschalenförmige Aufnahme für den Mehrkammermischbehälter (20) sowie Haltemittel (35) für den Mehrkammermischbehälter (20), um diesen in seiner Lage zu fixieren und einen in axialer Richtung beweglichen Ausdrückstempel (32). Der Ausdrückstempel (32) weist an seiner Hinterseite die Griffplatte (31) auf und eine Verdickung (33) oder einen Ring (33), um die axiale Bewegung des Ausdrückstempels (32) zu begrenzen. An der Spitze des Ausdrückstemples (32) befindet sich die Zentrierspitze (34), welche auf dem Boden (8) des Stülpbehälters (5) aufliegt, um diesen in Richtung Entnahmebehälter (16) bzw. in Richtung des Entnahmebereichs (18) des Entnahmebehälters (16) zu verschieben und dabei den Stülpbehälter (5) immer weiter einzustülpen. Figur 3 zeigt nun den Zustand, wo der Stülpbehälter (5) vollständig in den Entnahmebehälter (16) gestülpt worden ist und der Ausdrückstempel (32) bis zur Verdickung (33) oder bis zum Ring (33) eingedrückt ist. Die Zentrierspitze (34) des Ausdrückstempels (32) ist bis zum Entnahmebereich (18) des Entnahmebehälters (16) vorgeschoben und die Wandung (2) des Stülpbehälters (5) liegt an der Wandung (12) des Entnahmebehälters (16). Die Flüssigkeit (3), welche sich im Stülpbehälter (5) befunden hatte, konnte nach dem Durchdrücken der Siegelfolien (1 und 11) in den Entnahmebehälter (16) fließen und die Komponente (13) des Entnahmebehälters (16) lösen. Diese frisch hergestellte Lösung konnte kontinuierlich während der Fortsetzung des Drückvorgangs aus dem Entnahmebereich (18) und durch den Verschluß (15) des Entnahmebehälters (16) über eine Kanüle (36) ausfließen. Figur 3 zeigt den Zustand, wo der Mehrkammermischbehälter (20) vollständig entleert ist. Der Ausdrückstempel (32) kann nun aus dem eingestülpten Mehrkammermischbehälter (20) herausgezogen, der Mehrkammermischbehälter (20) aus der Vorrichtung (30) entnommen und entsorgt werden.
**Figur 4**
   Figur 4 zeigt einen Mehrkammermischbehälter (20) bestehend aus einem Stülpbehälter (5) und einem Entnahmebehälter (16), welche dichtend miteinander verbunden sind, eingelegt in eine Vorrichtung (30) zur Aufnahme des Mehrkammermischbehälters (20). Die Vorrichtung (30) umfasst eine halbschalenförmige Aufnahme für den Mehrkammermischbehälter (20) sowie Haltemittel (35) für den Mehrkammermischbehälter (20), um diesen in seiner Lage zu fixieren und einen in axialer Richtung beweglichen Ausdrückstempel (32). Der Ausdrückstempel (32) weist an seiner Hinterseite die Griffplatte (31) auf und eine Verdickung (33) oder einen Ring (33), um die axiale Bewegung des Ausdrückstempels (32) zu begrenzen. An der Spitze des Ausdrückstempels (32) befindet sich die Zentrierspitze (34), welche auf dem Boden (8) des Stülpbehälters (5) aufliegt, um diesen in Richtung Entnahmebehälter (16) bzw. in Richtung des Entnahmebereichs (18) des Entnahmebehälters (16) zu verschieben und dabei den Stülpbehälter (5) immer weiter einzustülpen. Figur 4 zeigt ferner den Zustand, wo der Stülpbehälter (5) etwas mehr als die Hälfte seiner Höhe eingestülpt worden ist, indem auf die Griffplatte (31) des Ausdrückstempels (32) ein Druck ausgeübt wird, der durch die Zentrierspitze (34) des Ausdrückstempels (32) auf den Boden (8) des Stülpbehälters (5) übertragen wird und dadurch den Boden gleichmäßig in axialer Richtung nach vorne schiebt und dabei den Stülpbehälter (5) einstülpt. Damit bei dieser Druckausübung der Mehrkammermischbehälter (20) in der Vorrichtung (30) nicht verrutscht, liegen die miteinander verbundenen (verklebten oder verschweißten) Nuten (7 und 17) in dem Haltemittel (35), welches als Aussparung ausgebildet ist. Ferner ist zu erkennen, dass der Boden (8) des Stülpbehälters (5) mittig eine Durchdrücknase (4) oder Einwölbung (4) oder Verdickung (4) aufweist, welche bereits die Siegelfolie (1) des Stülpbehälters (5) als auch die Siegelfolie (11) des Entnahmebehälters (16) durchdrückt hat, so dass die Flüssigkeit (3) aus dem Stülpbehälter (5) in den Entnahmebehälter (16) übertreten konnte, um die Komponente (13) des Entnahmebehälters (16) zu lösen. Die so entstandene Lösung kann durch oder aus dem Entnahmebereich (18) des Entnahmebehälters (16) mittels einer Nadel oder Kanüle oder auch nadelfrei entnommen werden.
**Figur 5**
   Figur 5 zeigt als Komponentenbehälter einen Zwischenbehälters (29), der ca. zur Hälfte mit einem Feststoff (23) oder einer Flüssigkeit (23) gefüllt ist. Die Oberseite (25) des Zwischenbehälters (29) ist mit einer Siegelfolie (21) verschlossen, so dass der Inhalt des Zwischenbehälters (29) aseptisch, steril und keimfrei beleibt. Die Siegelfolie (21) ist so ausgestaltet, dass sie durch den auf den Boden (8) des Stülpbehälters (5) ausgeübten Druck durchstoßen werden kann. Entlang des Randes der Oberseite (25) des Zwischenbehälters (29) verläuft eine Wulst oder Nut (27), welche zum dichtenden und/oder stoffschlüssigen Zusammenfügen der Komponentenbehälter vorteilhaft ist und auch als Mittel zum Festhalten in der Vorrichtung (30) zur Aufnahme des Mehrkammermischbehälters (20) dient. Die Unterseite (26) des Zwischenbehälters (29) ist mit einer Siegelfolie (24) verschlossen, so dass der Inhalt des Zwischenbehälters (29) aseptisch, steril und keimfrei beleibt. Die Siegelfolie (24) ist so ausgestaltet, dass sie durch den auf den Boden (8) des Stülpbehälters (5) ausgeübten Druck durchstoßen werden kann. Entlang des Randes der Unterseite (26) des Zwischenbehälters (29) verläuft eine Wulst oder Nut (28), welche zum dichtenden und/oder stoffschlüssigen Zusammenfügen der Komponentenbehälter vorteilhaft ist und auch als Mittel zum Festhalten in der Vorrichtung (30) zur Aufnahme des Mehrkammermischbehälters (20) dient. Die Siegelfolien (21 und 24) sind derart ausgestaltet, dass sie durch den auf den Boden (8) des Stülpbehälters (5) ausgeübten Druck durchstoßen werden können. Die Wandung (22) des Zwischenbehälters (29) kann starr oder stülpbar oder zum Teil starr und zum Teil stülpbar ausgestaltet sein. Soll der Zwischenbehälter (29) nicht in den Entnahmebehälter (16) gestülpt werden können, dann ist die Wandung (22) aus einem festen Material, welches den Drücken auf den Boden (8) des Stülpbehälters (5) standhalten kann, ohne deformiert zu werden. Soll hingegen der Zwischenbehälter (29) in den Entnahmebehälter (16) stülpbar sein, dann ist die Wandung (22) des Zwischenbehälters (29) flexibel und stülpbar ausgestaltet und kann auch Rastlinien aufweisen. Wird der Zwischenbehälter (29) stülpbar ausgestaltet, so besteht er vorzugsweise aus demselben Material wie der Stülpbehälter (5) und weist auch vorzugsweise die gleiche Art und Ausgestaltung von Rastlinien auf. Soll hingegen der Zwischenbehälter (29) nur teilweise stülpbar sein, so ist seine Wandung (22) bis zu der Höhe stülpbar ausgestaltet, bin wohin der Zwischenbehälter (29) stülpbar sein soll und darüber hinaus besteht die Wandung (22) aus einem festen nicht stülpbaren Material.
**Figur 6**
   Figur 6 zeigt einen Mehrkammermischbehälter (20) bestehend aus einem Stülpbehälter (5), einem Zwischenbehälter (29) und einem Entnahmebehälter (16), welche dichtend und aseptisch miteinander verbunden sind. Von oben schaut man in die Aussparung (6) oder Ausformung (6) zur Aufnahme des Ausdrückstempels (32) der Vorrichtung (30). Der Boden (8) des Stülpbehälters (5) weist eine Durchdrücknase (4) oder Einwölbung (4) oder Verdickung (4) auf, welche in Richtung Entnahmebehälter (16) zeigt. Der Stülpbehälter (5) ist mit einer Flüssigkeit (3) teilweise gefüllt. Die Wandung (2) des Stülpbehälters (5) weist an der Oberseite (9) eine umlaufende Wulst oder Nut (7) auf, welche stoffschlüssig mit der Wulst oder Nut (27) der Oberseite (25) des Zwischenbehälters (29) verbunden ist. Die stirnseitig angebrachte Siegelfolie (1) des Stülpbehälters (5) liegt über der Siegelfolie (21) des Zwischenbehälters (29). Der Zwischenbehälter (29) ist von der Wandung (22) umgeben, welche am Rand der Unterseite (26) eine weitere Wulst oder Nut (28) aufweist. Zudem befindet sich an der Unterseite (26) eine weitere Siegelfolie (24), wodurch der Inhalt (23) des Zwischenbehälters (29) keimfrei und steril verschlossen ist. Die Wulst oder Nut (28) ist dichtend und stoffschlüssig mit der Wulst oder Nut (17) des Entnahmebehälters (16) verbunden. Unter der Siegelfolie (24) des Zwischenbehälters (29) befindet sich die Siegelfolie (11) des Entnahmebehälters (16). Der Entnahmebehälter (16) ist mit einer Flüssigkeit (13) oder einem Feststoff (13) teilweise gefüllt und von der Wandung (12) umgeben. An der Unterseite weist der Entnahmebehälter (16) einen Verschluß (15) und einen Entnahmebereich (18) auf, durch den oder aus dem die frisch hergestellte Lösung aus der Komponente des Stülpbehälters (5) und der Komponente des Zwischenbehälters (29) und der Komponente des Entnahmebehälters (16) mittels einer Nadel oder Kanüle oder auch nadelfrei entnommen werden kann. Der Mehrkammermischbehälter (20) ist so aufgebaut, dass der Stülpbehälter (5) die gleiche Höhe wie der Zwischenbehälter (29) und der Entnahmebehälter (16) zusammen haben, oder er ist sogar noch etwas größer, so dass der Stülpbehälter (5) vollständig in den Zwischenbehälter (29) und den Entnahmebehälter (16) gestülpt werden kann.
**Figur 7**

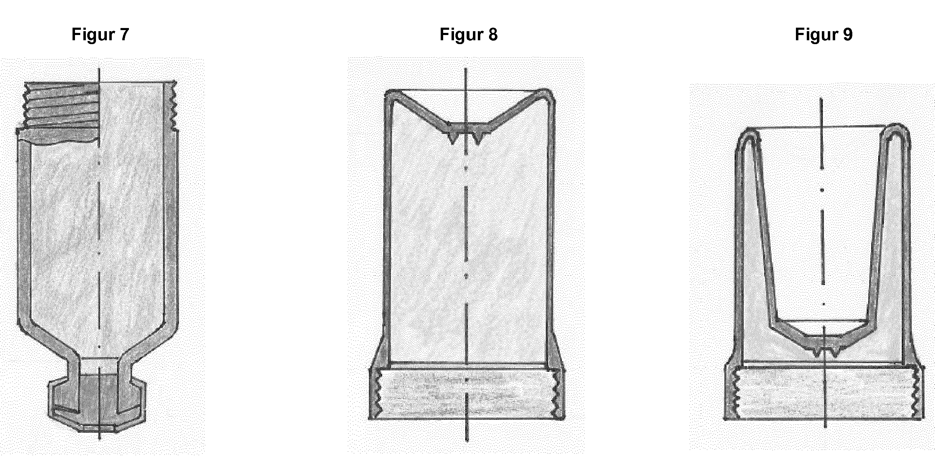

   Figur 7 zeigt einen Entnahmebehälter (16) eines erfindungsgemäßen Mehrkammermischbehälters (20) mit einen Gewinde zum dichtenden Verbinden mit einem Stülpbehälter (5). Der zugehörige Stülpbehälter (5) ist in Figur 8 in gefüllter und nicht gestülpter Form und in Figur 9 in eingestülpter Form dargestellt.
**Figur 8**
   Figur 8 zeigt einen Stülpbehälter (5) in gefüllter und nicht gestülpter Form mit einem Innengewinde zum formschlüssigen und dichtenden Verbinden mit einem Entnahmebehälter (16) wie in Figur 7 gezeigt.
**Figur 9**
   Figur 9 zeigt einen Stülpbehälter (5) in weitgehend entleerter und gestülpter Form mit einem Innengewinde zum formschlüssigen und dichtenden Verbinden mit einem Entnahmebehälter (16) wie in Figur 7 gezeigt.
**Figur 10**

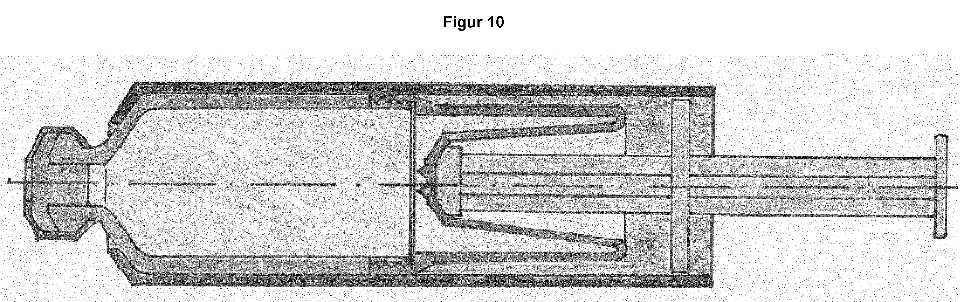

   Figur 10 zeigt einen Mehrkammermischbehälter (20) bestehend aus einem Stülpbehälter (5) wie in Figur 8 gezeigt und einem Entnahmebehälter (16) wie in Figur 7 gezeigt, dichtend verbunden über ein Gewinde wie bei einem Kugelschreiber, so dass ein glatter Außenmantel mit konstantem Außendurchmesser entsteht. Bei dieser Ausführungsform ist keine Nut oder Wulst vorhanden und der Mehrkammermischbehälter (20) kann aufgrund seines zylinderförmigen Außenmantels in eine rohrförmige Vorrichtung eingeschoben werden, so dass der Entnahmebereich des Entnahmebehälters (16) aus der rohrförmigen Vorrichtung herausragt und der Mehrkammermischbehälter (20) mittels eines Ausdrückstempels (32) entleert werden kann.
**Figur 11**
   Bei einer Vorrichtung, welche einem Aufbau entspricht wie in Figur 21 oder 22 von WO 2010051369 A1 gezeigt, wurde ein Dorn mit einem Durchmesser von 9,9 mm in einen Zylinder mit einem Innendurchmesser von 10,0 mm eingeführt, um eine Membran entsprechend der Membran 250 in den Figuren 21 und 22 von WO 2010051369 A1 zu durchstoßen und danach zu überwinden. Verwendet wurde ein kommerziell verfügbares Element eines Portsystem der Firma B.Braun Melsungen AG. Die Membran entsprechend der Membran 250 in den Figuren 21 und 22 von WO 2010051369 A1 wies eine Dicke von 0,2 mm auf. Eine zweite Membran entsprechend der Membran 250' in den Figuren 21 und 22 von WO 2010051369 A1 war nicht vorhanden. Figur 11 zeigt den Druckverlauf in Newton (N) über die vom Dorn zurückgelegte Wegstrecke (in mm) bei einer kontinuierlichen Geschwindigkeit von 200mm/min, wobei nach ca. 4 mm Wegstrecke und einem Druck von ca. 40N die Membran durchstoßen wird aber danach ein Druck von über 240N aufzuwenden ist, um die durchstoßene und an die Innenwand des Zylinders gedrückte Membran mit dem Dorn zu überwinden. Es ist deutlich zu erkennen, dass nach dem Überwinden der Membran nach ca. 8 mm ein erster und nach 11 mm Wegstrecke ein weiterer deutlicher und schneller Druckabfall auftritt, der zu einer unkontrollierten Axialbewegung des Kolbens und so an einen Patienten erhebliche Verletzungen verursachen kann. Ferner ist bei einer handbetätigten Vorrichtung zum Entleeren eines Behälters wie er zur Bestimmung des in Figur 12 gezeigten Druckverlaufs eingesetzt wurde und welcher den Behältern gemäß Figuren 21 und 22 von WO 2010051369 A1 entspricht, der Druckabfall nicht zu kontrollieren, so dass zwingend zumindest eine mechanische Vorrichtung zum Entleeren eingesetzt werden muss, was unpraktisch ist und zusätzliche Kosten verursacht und eine weitere Störquelle darstellen kann. Die zweite Linie in Figur 11 zeigt den Druckverlauf bei einem Wiederholdungsversuch mit gleichem Versuchsaufbau (Doppelbestimmung), um die Aussagekraft des Versuchs zu steigern.
**Figur 12**
   Bei einer Vorrichtung, welche einem Aufbau entspricht wie in Figur 21 oder 22 von WO 2010051369 A1 gezeigt, wurde ein Dorn mit einem Durchmesser von minimal unter 6,0 mm in einen Zylinder mit einem Innendurchmesser von 6,0 mm eingeführt, um eine Membran entsprechend der Membran 250 in den Figuren 21 und 22 von WO 2010051369 A1 zu durchstoßen und danach zu überwinden. Verwendet wurde ein kommerziell verfügbares Element eines Portsystem der Firma B.Braun Melsungen AG. Die Membran entsprechend der Membran 250 in den Figuren 21 und 22 von WO 2010051369 A1 wies eine Dicke von 0,2 mm auf. Eine zweite Membran entsprechend der Membran 250' in den Figuren 21 und 22 von WO 2010051369 A1 war nicht vorhanden. Figur 12 zeigt den Druckverlauf in Newton (N) über die vom Dorn zurückgelegte Wegstrecke (in mm) bei einer kontinuierlichen Geschwindigkeit von 200mm/min, wobei nach ca. 6 mm Wegstrecke die maximale Kraft von etwas über 40N erreicht wird. Es ist deutlich zu erkennen, dass nach dem Überwinden der Membran nach ca. 6 - 7 mm Wegstrecke ein deutlicher und schneller Druckabfall auftritt, der aber längst nicht so stark ist wie im vorigen Beispiel zu Figur 11 gezeigt, da der Dorn nicht durch an der Wandung anliegende Membranteile geklemmt wird. Die zweite Linie in Figur 12 zeigt den Druckverlauf bei einem Wiederholdungsversuch mit gleichem Versuchsaufbau (Doppelbestimmung), um die Aussagekraft des Versuchs zu steigern.
**Figur 13** zeigt die im den Beispielen 3 und 4 verwendeten Dorne (links: 6mm, rechts: 9,9mm). In Beispiel 11 wurde der Dorn mit 9,9 mm Durchmesser verwendet. Die zugehörige damit gemessene Druckkurve ist in Figur 11 dargestellt. In Beispiel 12 wurde der Dorn mit 6 mm Durchmesser verwendet. Die zugehörige damit gemessene Druckkurve ist in Figur 12 dargestellt.
**Figur 14** zeigt den 9,9mm Dorn in Einsteckposition.
**Figur 15** zeigt den Apparateaufbau mit dem Portelement vor dem Durchstechen.
**Figur 16** zeigt das Portelement vor dem Durchstechen.
**Figur 17** zeigt das Portelement nach dem Durchstechen.

### Beispiele

### Beispiel 1

Ein Stülpbehälter mit einem Gesamtvolumen 10 ml enthaltend 6 ml isotonische Natriumchloridlösung wird mit einem Entnahmebehälter mit einem Gesamtvolumen von 10 ml enthaltend ein lyophilisiertes Bleomycinpräparat in einer Menge von 6 mg an den vorhandenen Fügerändern, die eine Nutung bzw. Spundung aufweisen, unter aseptischen Bedingungen in einer Laminarbox zusammengesteckt und durch Wärmeeinwirkung verschweißt, so dass sich die Siegelfolien der beiden Komponentenbehälter gegenüberliegen. Der so entstandene Mehrkammermischbehälter wird in eine karpulenartige Vorrichtung gelegt, die Vorrichtung geschlossen, und mit dem Ausdrückstempel der Vorrichtung mechanischer Druck auf den Boden des Stülpbehälters ausgeübt. Der Stülpbehälter wird nach und nach eingestülpt, und der innenliegende Durchdrückstempel durchdrückt die beiden Siegelfolien zwischen Stülpbehälter und Entnahmebehälter. Der durch das sich verkleinernde Volumen des Stülpbehälters entstehende Druck führt zu einem schnellen Einströmen der Natriumchloridlösung in den Entnahmebehälter und Auflösen des lyophilisierten Bleomycinderivates. Das Stülpen des Stülpbehälters wird durch umlaufende Rastlinien in der Wandung des Stülpbehälters erleichtert. Die Nut zwischen Stülpbehälter und Entnahmebehälter stoppt den Stülpvorgang. Die überschüssige Luft entweicht durch das druckgeregelte Auslassventil des Entnahmebehälters. Der Stülpvorgang ist beendet, wenn der Stülpbehälter vollständig in den Entnahmebehälter gestülpt wurde. Die Bleomycinlösung wird durch eine Kanüle durch den Stopfen in dem Entnahmebereich auf der Unterseite des Entnahmebehälters entnommen und in einen Tropfbeutel überführt.

### Beispiel 2

Ein Stülpbehälter mit einem Gesamtvolumen von 8 ml enthaltend 5 ml isotonische Natriumchloridlösung wird mit einem Zwischenbehälter mit einem Gesamtvolumen von 4 ml enthaltend 40 mg pulverförmiges Ibuprofen durch Kleben unter sterilen Bedingungen stoffschlüssig zusammengefügt. An die andere Seite des Zwischenbehälters wird ein Entnahmebehälter mit einem Gesamtvolumen von 8 ml durch Kleben unter sterilen Bedingungen stoffschlüssig angefügt, der 500 mg Vitamin C in pulverförmiger Form enthält. Der Stülpbehälter wird eingestülpt, und der innenliegende Ausdrückstempel durchdrückt die beiden Siegelfolien zwischen Stülp- und Zwischenbehälter und nachfolgend die beiden Siegelfolien zwischen Zwischen- und Entnahmebehälter. Der Stülpbehälter hat keine Rastlinien und wird vollständig in das Volumen von Zwischen- und Entnahmebehälter hineingestülpt. Der durch das sich verkleinernde Volumen des Stülpbehälters entstehende Druck führt zu einem schnellen Einströmen der Natriumchloridlösung in den Entnahmebehälter und Auflösen der pulverförmigen Stoffe. Die überschüssige Luft entweicht durch das druckgeregelte Auslassventil des Entnahmebehälters. Die Ibuprofenlösung wird mittels einer Nadel durch den Entnahmebereich in Form eines Septums entnommen und auf eine Spritze aufgezogen.

### Beispiel 3

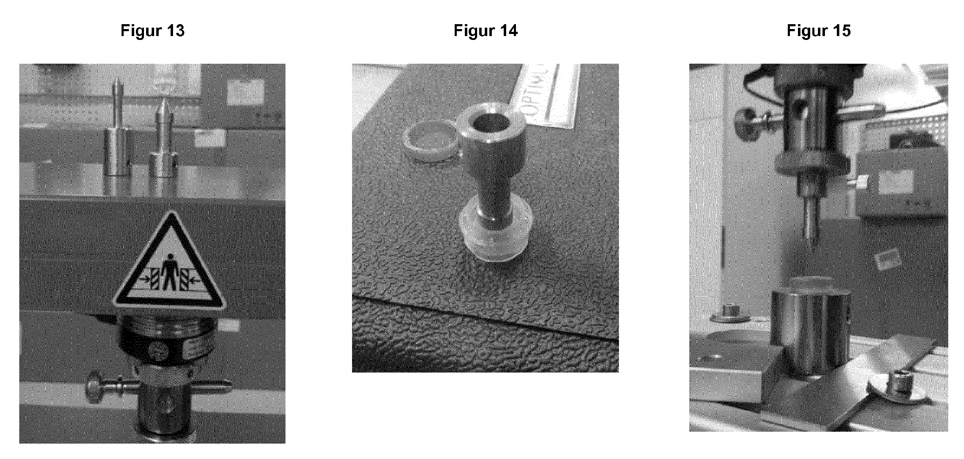

Die Figuren 13 - 17 zeigen den Versuchsaufbau schematisch. Mit Ausnahme des verwendeten Dorns ist der Aufbau für beide Versuche identisch.

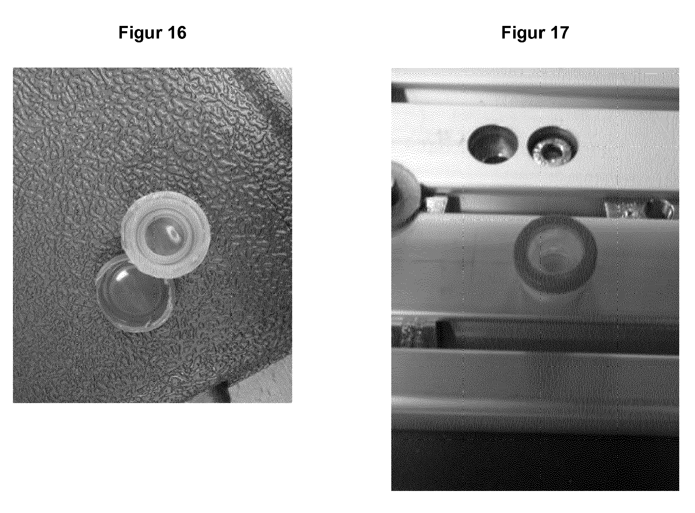

Figur 13 zeigt die verwendeten Dorne (links: 6mm, rechts: 9,9mm). Figur 14 zeigt den 9,9mm Dorn in Einsteckposition. Figur 15 zeigt den Apparateaufbau mit dem Portelement vor dem Durchstechen. Figur 16 zeigt das Portelement vor dem Durchstechen. Figur 17 zeigt das Portelement nach dem Durchstechen.

Der jeweilige Dorn (Figur A) wird in die Aufnahme einer handelsüblichen Zugprüfmaschine gesteckt und mit einem Stift gegen Herausrutschen gesichert (Figur 15). Das Portelement wird in eine Aufnahme eingelegt (Figur 15) und auf dem Tisch zentrisch zum dem Dorn ausgerichtet. Diese Position wird durch Anschläge fixiert. An der Zugprüfmaschine werden die Prüfbedingungen eingestellt. Die Verfahrwege wurden anhand eines manuell eingestochenen Dorns mit einem Meßschieber ermittelt (Figur 17). Es wurden gewählt:
Verfahrgeschwindigkeit: 200mm/min
Bedingung zum Abbruch: Erreichen einer definierten Weggrenze (Einstichtiefe)
Bedingung zum Start: Erreichen einer Vorkraft von 2N

Nach Einlegen des Portsystems in die Halterung wird das Programm gestartet. Der Dorn fährt mit einer definierten Geschwindigkeit bis zum voreingestellten Weg. Nach dessen Erreichen fährt die Maschine automatisch wieder in die Ausgangsposition zurück.

### Beispiel 4

Entspricht Beispiel 3 nur dass ein Dorn mit 6 mm Durchmesser verwendet wurde.

## Patentansprüche

1. Mehrkammermischbehälter (20) umfassend oder bestehend aus einem Stülpbehälter (5) mit einer Wandung (2) und einem Entnahmebehälter (16), wobei der Stülpbehälters (5) mit einer Flüssigkeit (3) gefüllt ist und stirnseitig mit einer durchdrückbaren Siegelfolie (1) verschlossen ist und dessen Boden (8) in Richtung der Siegelfolie (1) drückbar ist und der Entnahmebehälter (16) mit einer Flüssigkeit oder einem Feststoff (13) gefüllt ist und auf der Oberseite (19) mit einer durchdrückbaren Siegelfolie (11) verschlossen ist und auf der Unterseite einen Entnahmebereich (18) aufweist und Stülpbehälter (5) und Entnahmebehälter (16) dichtend miteinander verbunden sind dadurch charakterisiert, dass
die Wandung (2) des Stülpbehälters (5) nach innen stülpbar ist.

2. Mehrkammermischbehälter (20) gemäß Anspruch 1, wobei die mit der durchdrückbaren Siegelfolie (1) verschlossene Stirnseite (19) des Stülpbehälters (5) mit der mit der durchdrückbaren Siegelfolie (11) verschlossenen Oberseite (9) des Entnahmebehälters (16) dichtend verbunden sind.

3. Mehrkammermischbehälter (20) gemäß Anspruch 1 oder 2, wobei die Außenseite des Stülpbehälters (5) in Höhe der Siegelfolie (1) eine Nut (7), eine Wulst (7), ein Gewinde oder eine Einbuchtung aufweist und/oder der Entnahmebehälter (16) in Höhe der Siegelfolie (11) eine Nut (17), eine Wulst (17), ein Gewinde oder eine Einbuchtung aufweist.

4. Mehrkammermischbehälter (20) gemäß einem der Ansprüche 1 - 3, wobei der Boden (8) des Stülpbehälters (5) eine in Richtung der Siegelfolie (1) ausgebildete und mittig angeordnete Durchdrücknase (4) oder Einwölbung (4) oder Verdickung (4) aufweist oder der Boden konkav geformt ist.

5. Mehrkammermischbehälter (20) gemäß Anspruch 4, wobei die Durchdrücknase (4) oder die Einwölbung (4) oder die Verdickung (4) oder der konkav geformte Boden Kanäle, Rillen, Wellen, sternförmige Vertiefungen, Hügel, Noppen, Stifte oder andere Oberflächenunebenheiten aufweist.

6. Mehrkammermischbehälter (20) gemäß einem der Ansprüche 1 - 5, wobei die stülpbare Wandung (2) des Stülpbehälters (5) mit umlaufenden Rastlinien (10) versehen ist.

7. Mehrkammermischbehälter (20) gemäß einem der Ansprüche 1 - 6 bestehend aus dem Stülpbahälter (5) und dem Entnahmebehälter (16) und einem zwischen Stülpbehälter (5) und Entnahmebehälter (16) befindlichen Zwischenbehälter (29) gefüllt mit einer Flüssigkeit oder einem Feststoff, (23) wobei der Zwischenbehälter (29) an der Oberseite (25) und an der Unterseite (26) mit einer durchdrückbaren Siegelfolie (24) verschlossen ist und der Zwischenbehälter (29) dichtend mit dem Stülpbehälter (5) und dichtend mit dem Entnahmebehälter (16) verbunden ist.

8. Mehrkammermischbehälter (20) gemäß einem der Ansprüche 1 - 6 bestehend aus dem Stülpbehälter (5) und dem Entnahmebehälter (16) und zwei zwischen Stülpbehälter (5) und Entnahmebehälter (16) befindlichen Zwischenbehältern (29) jeweils gefüllt mit einer Flüssigkeit oder einem Feststoff (23), wobei beide Zwischenbehälter (29) an der Oberseite (25) und an der Unterseite (26) mit einer durchdrückbaren Siegelfolie (24) verschlossen sind und beide Zwischenbehälter (29) dichtend miteinander verbunden sind und der eine Zwischenbehälter (29) dichtend mit dem Stülpbehälter (5) und der andere Zwischenbehälter (29) dichtend mit dem Entnahmebehälter (16) verbunden ist.

9. Mehrkammermischbehälter (20) gemäß einem der Ansprüche 1 - 8, wobei der Mehrkammermischbehälter (20) bestehend aus Stülpbehälter (5) und Entnahmebehälter (16) außen entlang der Verbindungslinie zwischen Stülpbehälter (5) und Entnahmebehälter (16) eine Nut (7, 17) aufweist oder der Mehrkammermischbehälter (20) bestehend aus Stülpbehälter (5), Zwischenbehälter (29) und Entnahmebehälter (16) außen entlang der Verbindungslinie zwischen Stülpbehälter (5) und Zwischenbehälter (29) und/oder zwischen Zwischenbehälter (29) und Entnahmebehälter (16) eine Nut (7, 17, 27, 28) aufweist oder der Mehrkammermischbehälter (20) bestehend aus Stülpbahalter (5), zwei Zwischenbehältern (29) und Entnahmebehälter (16) außen entlang der Verbindungslinie zwischen Stüipbehälter (5) und dem einen Zwischenbehälter (29) und/oder zwischen den beiden Zwischenbehältern (29) und/oder zwischen dem anderen Zwischenbehälter (29) und Entnahmebehälter (16) eine Nut (7, 17, 27, 28) aufweist.

10. Mehrkammermischbehälter (20) gemäß Anspruch 7, wobei zur dichtenden Verbindung zwischen Stülpbehälter (5) und Zwischenbehälter (29) und zwischen Zwischenbehälter (29) und Entnahmebehälter (16), der Stülpbehälter (5), der Zwischenbehälter (29) und der Entnahmebehälter (16) in Höhe der Siegelfolien (1, 11, 21) eine Nut (7, 17, 27, 28), eine Wulst, ein Gewinde oder eine Einbuchtung aufweisen.

11. Mehrkammermischbehälter (20) gemäß Anspruch 8, wobei zur dichtenden Verbindung zwischen Stülpbehälter (5) und dem einen Zwischenbehälter (29) und zwischen dem einen und dem anderen Zwischenbehälter (29) und zwischen dem anderen Zwischenbehälter (29) und dem Entnahmebehälter (16), der Stülpbehälter (5), der eine Zwischenbehälter (29), der andere Zwischenbehälter (29) und der Entnahmebehälter (16) in Höhe der Siegelfolien (1, 11, 21) eine Nut (7, 17, 27, 28), eine Wulst, ein Gewinde oder eine Einbuchtung aufweisen.

12. Mehrkammermischbehälter (20) gemäß einem der Ansprüche 1 - 11, wobei der Stülpbehälter (5) vollständig mit einer Flüssigkeit (3) gefüllt ist.

13. Kit umfassend mindestens einen eine Wandung (2) aufweisenden Stülpbehälter (5), der stirnseitig mit einer durchdrückbaren Siegelfolie (1) verschlossen ist und dessen Boden (8) in Richtung der Siegelfolie (1) drückbar ist und die Wandung (2) nach innen stülpbar ist und mindestens einen Entnahmebehälter (16), der auf der Oberseite (19) mit einer durchdrückbaren Siegelfolie (11) verschlossen ist und auf der Unterseite einen Entnahmebereich (18) aufweist, wobei Stülpbehälter (5) und Entnahmebehälter (16) dichtend miteinander verbindbar sind.

14. Kit gemäß Anspruch 13, des weiteren umfassend mindestens einen Zwischenbehälter (29), der an der Oberseite (25) und an der Unterseite (26) mit einer durchdrückbaren Siegelfolie (24) verschlossen ist, wobei der Zwischenbehälter (29) dichtend mit dem Stülpbehälter (5) und dichtend mit dem Entnahmebehälter (16) verbindbar ist.

15. Methode zur aseptischen Herstellung einer Lösung aus mindestens zwei Komponenten, charakterisiert durch das Einlegen eines Mehrkammermischbehälters (20) gemäß einem der Ansprüche 1 - 14 in eine Vorrichtung (30) umfassend eine halbschalenförmige Aufnahme für den Mehrkammermischbehälter (20), Haltemittel (35) für den Mehrkammermischbehälter (20) und einen in axialer Richtung beweglichen Ausdrückstempel (32), Ausübung von Druck durch den Ausdrückstempel (32) auf den Boden (8) des Stülpbehälters (5), Drücken des Bodens (8) des Stülpbehälters (5) in Richtung des Entnahmebehälters (16), wobei sich die Wandung (2) des Stülpbehälters (5) kontinuierlich nach innen stülpt und mit forschreitendem Drücken die Siegelfolien (1, 11) durchdrückt werden und Vermischung der mindestens zwei Komponenten.

## Claims

1. Multi-chamber mixing container (20) comprising or consisting of an introversion container (5) having a wall (2) and a withdrawal container (16), wherein the introversion container (5) is filled with a liquid (3) and sealed with a pierceable sealing foil (1) on the front side and its bottom is (8) pushable in the direction of the sealing foil (1) and the withdrawal container (16) is filled with a liquid or a solid (13) and sealed on the top side (19) with a pierceable sealing foil (11) and has a withdrawal area (18) at the bottom side and introversion container (5) and withdrawal container (16) are connected with each other in a sealing manner, **characterized in that** the wall is introversible to the inside.

2. Multi-chamber mixing container (20) according to claim 1, wherein the front side (19) of the introversion container (5) sealed with the pierceable sealing foil (1) and the top side (9) of the withdrawal container (16) sealed with the pierceable sealing foil (11) are connected with each other in a sealing manner.

3. Multi-chamber mixing container (20) according to claim 1 or 2, wherein the outer face of the introversion container (5) has a groove (7), a bead (7), a thread or a recess at the level of the sealing foil (1) and/or the withdrawal container (16) has a groove (17), a bead (17), a thread or a recess at the level of the sealing foil (11).

4. Multi-chamber mixing container (20) according to any one of claims 1 - 3, wherein the bottom (8) of the introversion container (5) has a piercing nose (4) or protrusion (4) or bulge (4) formed in the direction of the sealing foil (1) and positioned centrally or the bottom is shaped concave.

5. Multi-chamber mixing container (20) according to claim 4, wherein the piercing nose (4) or the protrusion (4) or the bulge (4) or the concavely shaped bottom has canals, furrows, corrugations, star-shaped indentations, mounds, knobs, pins or other surface irregularities.

6. Multi-chamber mixing container (20) according to any one of claims 1 - 5, wherein the introversible wall (2) of the introversion container (5) has circumferential notch lines (10).

7. Multi-chamber mixing container (20) according to any one of claims 1 - 6, consisting of the introversion container (5) and the withdrawal container (16) and an intermediate container (29) filled with a liquid or a solid (23) and located in between introversion container (5) and withdrawal container (16), wherein the intermediate container (29) is sealed on the top side (25) and on the bottom side (26) with a pierceable sealing foil (24) and the intermediate container (29) is connected with the introversion container (5) in a sealing manner and with the withdrawal container (16) in a sealing manner.

8. Multi-chamber mixing container (20) according to any one of claims 1 - 6, consisting of the introversion container (5) and the withdrawal container (16) and two intermediate containers (29) each filled with a liquid or a solid (23) and located in between introversion container (5) and withdrawal container (16), wherein both intermediate containers (29) are sealed on the top side (25) and on the bottom side (26) with a pierceable sealing foil (24) and both intermediate containers (29) are connected with each other in a sealing manner and the first intermediate container (29) is connected with the introversion container (5) in a sealing manner and the other intermediate container (29) is connected with the withdrawal container (16) in a sealing manner.

9. Multi-chamber mixing container (20) according to any one of claims 1 - 8, wherein the multi-chamber mixing container (20) consisting of introversion container (5) and withdrawal container (16) has on the outside along the connecting line between introversion container (5) and withdrawal container (16) a groove (7, 17) or the multi-chamber mixing container (20) consisting of introversion container (5), intermediate container (29) and withdrawal container (16) has on the outside along the connecting line between introversion container (5) and intermediate container (29) and/or between intermediate container (29) and withdrawal container (16) a groove (7, 17, 27, 28) or the multi-chamber mixing container (20) consisting of introversion container (5), two intermediate containers (29) and withdrawal container (16) has on the outside along the connecting line between introversion container (5) and the first intermediate container (29) and/or between the both intermediate containers (29) and/or between the other intermediate container (29) and withdrawal container (16) a groove (7, 17, 27, 28).

10. Multi-chamber mixing container (20) according to claim 7, wherein for the sealing connection between introversion container (5) and intermediate container (29) and between intermediate container (29) and withdrawal container (16), the introversion container (5), the intermediate container (29) and the withdrawal container (16) have a groove (7, 17, 27, 28), a bead, a thread or a recess at the level of the sealing foils (1, 11, 21).

11. Multi-chamber mixing container (20) according to claim 8, wherein for the sealing connection between introversion container (5) and the first intermediate container (29) and between the first and the other intermediate container (29) and between the other intermediate container (29) and the withdrawal container (16), the introversion container (5), the first intermediate container (29), the other intermediate container (29) and the withdrawal container (16) have a groove (7, 17, 27, 28), a bead, a thread or a recess at the level of the sealing foils (1, 11, 21).

12. Multi-chamber mixing container (20) according to any one of claims 1 - 11, wherein the introversion container (5) is filled completely with a liquid (3).

13. Kit comprising at least one introversion container (5) having a wall (2) and that is sealed with a pierceable sealing foil (1) on the front side and its bottom (8) is pushable in the direction of the sealing foil (1) and the wall (2) is introversible to the inside and at least one withdrawal container (16) that is sealed on the top side (19) with a pierceable sealing foil (11) and has a withdrawal area (18) at the bottom side, wherein introversion container (5) and withdrawal container (16) are connectable with each other in a sealing manner.

14. Kit according to claim 13, further comprising at least one intermediate container (29) that is sealed on the top side (25) and on the bottom side (26) with a pierceable sealing foil (24), wherein the intermediate container (29) is connectable with the introversion container (5) in a sealing manner and with the withdrawal container (16) in a sealing manner.

15. Method for the aseptic preparation of a solution from at least two components, **characterized by** the insertion of a multi-chamber mixing container (20) according to any one of claims 1 - 14 in a device (30) comprising a receptacle in the shape of a half-shell for the multi-chamber mixing container (20), mounting means (35) for the multi-chamber mixing container (20) and an expression plunger (32) movable in the axial direction, exertion of pressure by the expression plunger (32) onto the bottom (8) of the introversion container (5), pushing the bottom (8) of the introversion container (5) in the direction of the withdrawal container (16), whereat the wall (2) of the introversion container (5) is continuously introverting to the inside and with onward pushing the sealing foils (1, 11) are pierced and mixing of the at least two components.

## Revendications

1. Une cuve de mélange à chambres multiples (20) comprenant ou constitué par un conteneur à enfiler (5) avec une paroi (2) et un conteneur de prélèvement (16), où le conteneur à enfiler (5) est rempli avec un liquide (3) et fermé sur la face frontale avec un film perçable de scellage (1) et son fond (8) est enfonçable dans la direction du film de scellage (1) et le conteneur de prélèvement (16) est rempli avec un liquide ou un solide (13), et est fermé sur la face supérieure (19) avec un film perçable de scellage (11) et présente une zone de prélèvement (18) à la face inférieure et le conteneur à enfiler (5) et le conteneur de prélèvement (16) sont interconnectés de manière étanche, **caractérisée en ce que** la paroi (2) du conteneur (5) peut être enfilée vers l'intérieur.

2. La cuve de mélange à chambres multiples (20) selon la revendication 1, dans laquelle la face frontale (9) du conteneur à enfiler (5) fermée avec un film perçable de scellage (1) et la face supérieure (19) du conteneur à prélèvement (16) fermée avec un film perçable de scellage (11) sont connectées l'une à l'autre de manière étanche.

3. La cuve de mélange à chambres multiples (20) selon la revendication 1 ou 2, dans laquelle la face extérieure du conteneur à enfiler (5) a une rainure (7), un boudin (7), un filet ou une indentation au niveau du film de scellage (1) et/ou le conteneur de prélèvement (16) a une rainure (17), un boudin (17), un filet ou une indentation au niveau du film de scellage (11).

4. La cuve de mélange à chambres multiples (20) selon une quelconque des revendications 1 - 3, dans laquelle le fond (8) du conteneur à enfiler (5) a un nez perçant (4) ou une protrusion (4) ou une protubérance (4) formée dans la direction du film de scellage (1) et positionnée centralement, ou le fond a une forme concave.

5. La cuve de mélange à chambres multiples (20) selon la revendication 4, dans laquelle le nez perçant (4) ou la protrusion (4) ou la protubérance (4) ou le fond à forme concave a des canaux, des sillons, des ondulations, des indentations à forme d'étoile, des monticules, des poignées, des broches ou d'autres irrégularités de surface.

6. La cuve de mélange à chambres multiples (20) selon une quelconque des revendications 1 - 5, dans laquelle la paroi à enfiler (2) du conteneur à enfiler (5) a des lignes d'encoche circonférentielles (10).

7. La cuve de mélange à chambres multiples (20) selon une quelconque des revendications 1 - 6 constituée par le conteneur à enfiler (5), le conteneur de prélèvement (16) et un conteneur intermédiaire (29) rempli avec un liquide ou un solide (23) et positionné entre le conteneur à enfiler (5) et le conteneur de prélèvement (16), où le conteneur intermédiaire (29) est fermé sur la face supérieure (25) et sur la face inférieure (26) avec un film perçable de scellage (24) et le conteneur intermédiaire (29) est connecté avec le conteneur à enfiler (5) de manière étanche et avec le conteneur de prélèvement (16) de manière étanche.

8. La cuve de mélange à chambres multiples (20) selon une quelconque des revendications 1 - 6 constituée par le conteneur à enfiler (5), le conteneur de prélèvement (16) et deux conteneurs intermédiaires (29) chacun rempli avec un liquide ou un solide (23) et positionnés entre le conteneur à enfiler (5) et le conteneur de prélèvement (16), où les deux conteneurs intermédiaires (29) sont fermés sur la face supérieure (25) et sur la face inférieure (26) avec un film perçable de scellage (24) et les deux conteneurs intermédiaires (29) sont connectés l'un à l'autre de manière étanche et le premier conteneur intermédiaire (29) est connecté avec le conteneur à enfiler (5) de manière étanche et l'autre conteneur intermédiaire (29) est connecté avec le conteneur de prélèvement (16) de manière étanche.

9. La cuve de mélange à chambres multiples (20) selon une quelconque des revendications 1 - 8, où la cuve de mélange à chambres multiples (20) constituée par le conteneur à enfiler (5) et le conteneur de prélèvement (16) a à l'extérieur le long de la ligne de connexion entre le conteneur à enfiler (5) et le conteneur de prélèvement (16) une rainure (7, 17), ou la cuve de mélange à chambres multiples (20) constituée par le conteneur à enfiler (5), le conteneur intermédiaire (29) et le conteneur de prélèvement (16) a à l'extérieur le long de la ligne de connexion entre le conteneur à enfiler (5) et le conteneur intermédiaire (29) et/ou entre le conteneur intermédiaire (29) et le conteneur de prélèvement (16) une rainure (7, 17, 27, 28), ou la cuve de mélange à chambres multiples (20) constituée par le conteneur à enfiler (5), deux conteneurs intermédiaires (29) et le conteneur de prélèvement (16) a à l'extérieur le long de la ligne de connexion entre le conteneur à enfiler (5) et le premier conteneur intermédiaire (29) et/ou entre les deux conteneurs intermédiaires (29) et/ou entre l'autre conteneur intermédiaire (29) et le conteneur de prélèvement (16) une rainure (7, 17, 27, 28).

10. La cuve de mélange à chambres multiples (20) selon la revendication 7, où le conteneur à enfiler (5), le conteneur intermédiaire (29) et le conteneur de prélèvement (16) ont une rainure (7, 17, 27, 28), un boudin, un filet ou une indentation au niveau des films de scellage (1, 11, 21) pour former la connexion étanche entre le conteneur à enfiler (5) et le conteneur intermédiaire (29) et entre le conteneur intermédiaire (29) et le conteneur de prélèvement (16).

11. La cuve de mélange à chambres multiples (20) selon la revendication 8, où le conteneur à enfiler (5), le premier conteneur intermédiaire (29), l'autre conteneur intermédiaire (29) et le conteneur de prélèvement (16) ont une rainure (7, 17, 27, 28), un boudin, un filet ou une indentation au niveau des films de scellage (1, 11, 21) pour former la connexion étanche entre le conteneur à enfiler (5) et le premier conteneur intermédiaire (29) et entre le premier et l'autre conteneur intermédiaire (29) et entre l'autre conteneur intermédiaire (29) et le conteneur de prélèvement (16).

12. La cuve de mélange à chambres multiples (20) selon une quelconque des revendications 1 - 11, dans laquelle le conteneur à enfiler (5) est rempli complètement avec un liquide (3).

13. Un kit comprenant au moins un conteneur à enfiler (5) ayant une paroi (2) et étant fermé sur la face frontale avec un film perçable de scellage (1) et ayant un fond (8) enfonçable dans la direction du film de scellage (1) et dans lequel la paroi (2) peut être enfilée vers l'intérieur et au moins un conteneur de prélèvement (16) étant fermé sur la face supérieure (19) avec un film perçable de scellage (11) et ayant une zone de prélèvement (18) à la face inférieure, où le conteneur à enfiler (5) et le conteneur de prélèvement (16) sont interconnectés de manière étanche.

14. Le kit selon la revendication 13, comprenant en outre au moins un conteneur intermédiaire (29) qui est fermé sur la face supérieure (25) et sur la face inférieure (26) avec un film perçable de scellage (24), où le conteneur intermédiaire (29) est connectable avec le conteneur à enfiler (5) de manière étanche et avec le conteneur de prélèvement (16) de manière étanche.

15. Une méthode pour la préparation aseptique d'une solution á partir d'au moins deux ingrédients, **caractérisée par** :
insérer une cuve de mélange à chambres multiples (20) selon une quelconque des revendications 1 - 14 dans un dispositif (30) comprenant un réceptacle en forme de demi-coquille pour la cuve de mélange à chambres multiples (20), des moyens de maintien (35) pour la cuve de mélange à chambres multiples (20) et un piston (32) déplaçable dans la direction axiale par pression,
exercer de pression sur le fond (8) du conteneur à enfiler (5) par le piston (32), enfoncer le fond (8) du conteneur á enfiler (5) en direction du conteneur de prélèvement (16),
où la paroi (2) du conteneur à enfiler (5) est continuellement enfilée vers l'intérieur et suite à l'enfoncement ultérieur les films de scellage (1, 11) sont percés et les au moins deux ingrédients sont mélangés.
